(19) **Europäisches Patentamt / European Patent Office / Office européen des brevets**

(11) **EP 1 326 536 B1**

(12) **FASCICULE DE BREVET EUROPEEN**

(45) Date de publication et mention de la délivrance du brevet:
**10.12.2008 Bulletin 2008/50**

(21) Numéro de dépôt: **01980597.7**

(22) Date de dépôt: **17.10.2001**

(51) Int Cl.:
***A61B 8/15*** (2006.01)

(86) Numéro de dépôt international:
**PCT/FR2001/003208**

(87) Numéro de publication internationale:
**WO 2002/032316 (25.04.2002 Gazette 2002/17)**

(54) **PROCEDE ET DISPOSITIF NON INVASIF DE FOCALISATION D'ONDES ACOUSTIQUES**

NICHTINVASIVES GERÄT UND VERFAHREN ZUM FOKUSSIEREN VON SCHALLWELLEN

METHOD AND NON-INVASIVE DEVICE FOR FOCUSING ACOUSTIC WAVES

(84) Etats contractants désignés:
**AT BE CH CY DE DK ES FI FR GB GR IE IT LI LU MC NL PT SE TR**

(30) Priorité: **20.10.2000 FR 0013501**

(43) Date de publication de la demande:
**16.07.2003 Bulletin 2003/29**

(73) Titulaires:
• **Centre National de la Recherche Scientifique ( CNRS)**
  **75794 Paris Cedex 16 (FR)**
• **L'Université Paris VII (Denis Diderot)**
  **75251 Paris Cedex 05 (FR)**

(72) Inventeurs:
• **AUBRY, Jean-François**
  **F-92340 Bourg la Reine (FR)**

• **FINK, A., Mathias**
  **F-92190 Meudon (FR)**
• **TANTER, Mickael**
  **F-75006 Paris (FR)**
• **THOMAS, Jean-Louis**
  **F-94800 Villejuif (FR)**

(74) Mandataire: **Burbaud, Eric**
  **Cabinet Plasseraud**
  **52 rue de la Victoire**
  **75440 Paris Cedex 09 (FR)**

(56) Documents cités:
**US-A- 3 934 458      US-A- 5 207 214**
**US-A- 5 675 554**

**Description**

La présente invention est relative aux procédés et dispositifs non invasifs de focalisation d'ondes acoustiques, notamment ultrasonores.

Plus particulièrement, l'invention concerne un procédé non invasif de focalisation d'ondes acoustiques dans un milieu hétérogène dissipatif comprenant un milieu sensiblement homogène (par exemple, le cerveau) entouré au moins partiellement par une couche aberratrice dissipative (par exemple, le crâne) qui génère des aberrations dans la propagation des ondes acoustiques, les ondes acoustiques étant émises depuis l'extérieur de la couche aberratrice et focalisées dans le milieu sensiblement homogène.

Les procédés de ce type qui sont couramment utilisés ne permettent pas d'obtenir une bonne focalisation des ondes acoustiques à l'intérieur du milieu, et lorsque ces procédés sont utilisés dans des applications d'imagerie, ils ne permettent donc pas d'obtenir une bonne résolution et un bon contraste d'image lorsque les aberrations de propagation sont importantes, par exemple lorsqu'on réalise une échographie du cerveau depuis l'extérieur du crâne.

La présente invention a notamment pour but de pallier cet inconvénient.

A cet effet, il est décrit selon l'invention, un procédé de focalisation du genre en question comportant les étapes suivantes :

(a) une étape initiale de positionnement au cours de laquelle on fixe un nombre t supérieur à 2 de transducteurs acoustiques dans des positions prédéterminées à l'extérieur de la couche aberratrice, ces transducteurs étant en contact (direct ou indirect) avec ladite couche aberratrice et formant au moins :

- un réseau d'imagerie qui regroupe un nombre n compris entre 1 et t desdits transducteurs,
- et un réseau cible qui regroupe un nombre m compris entre 1 et t desdits transducteurs (ces deux réseaux peuvent être entièrement distincts, ou comporter certains transducteurs communs, ou encore comporter chacun la totalité des transducteurs susmentionnés),

(b) une étape d'apprentissage comprenant elle-même les sous étapes suivantes :

(b1) une sous-étape d'apprentissage de la focalisation du réseau d'imagerie sur le réseau cible, sous-étape au cours de laquelle :

(b11) on détermine des réponses impulsionnelles hri(t) du milieu hétérogène dissipatif, respectivement entre chaque transducteur i du réseau d'imagerie et plusieurs points de focalisation r situés sur la couche aberratrice en correspondance respective avec des transducteurs du réseau cible (cette détermination peut être faite par mesure directe si l'on fait émettre des impulsions acoustiques par les transducteurs du réseau cible, ou le cas échéant par mesure et calcul si l'on fait émettre des signaux acoustiques autres que des impulsions par les transducteurs du réseau cible, les valeurs ainsi mesurées et/ou calculées pouvant ensuite être le cas échéant corrigées par repropagation numérique pour simuler des transducteurs situés directement au contact de la couche aberratrice si les transducteurs ne sont pas au contact direct de la couche aberratrice), ces réponses impulsionnelles étant mémorisées sous forme numérique avec un certain échantillonnage temporel qui détermine un nombre p de composantes fréquentielles de la réponse impulsionnelle, de fréquences respectives $\omega k$, i étant un indice compris entre 1 et n qui désigne un transducteur du réseau d'imagerie, r étant un indice compris entre 1 et m qui désigne un point de focalisation correspondant à un transducteur du réseau cible et k étant un indice compris entre 1 et p qui désigne une composante fréquentielle,

(b12) à partir de ces réponses impulsionnelles, on calcule, pour chaque point de focalisation r correspondant à un transducteur du réseau cible, un ensemble de n signaux de référence temporels e'i(t,r), i variant entre 1 et n, tels que, si la paroi aberratrice était enlevée au voisinage du point de focalisation r, l'émission de ces signaux de référence par les différents transducteurs i du réseau d'imagerie génèrerait un signal prédéterminé (par exemple une impulsion acoustique) focalisé sur le point de focalisation r,

(b2) une sous-étape de focalisation en un nombre R de points de focalisation prédéterminés situés dans le milieu sensiblement homogène, d'indices q compris entre m+1 et m+R, cette sous-étape consistant à déterminer pour chacun de ces points de focalisation q, en s'éloignant pas à pas des points de focalisation 1 à m correspondant aux transducteurs du réseau cible, des signaux de référence e'i(t,q) à faire émettre par les différents transducteurs i du réseau d'imagerie pour générer une impulsion focalisée sur ledit point de focalisation q, les signaux de référence e'i(t,q) étant déterminés pour chaque point de focalisation q en procédant comme suit :

(b21) une première estimation de e'i(t,q), pour i allant de 1 à n, est calculée à partir d'au moins un signal de référence e'i(t,q0), q0 étant l'indice d'au moins un point de focalisation proche du point de focalisation q pour lequel le signal de référence a déjà été déterminé, ce calcul étant fait en utilisant une célérité moyenne des ondes acoustiques dans le milieu sensiblement homogène,

(b22) on fait émettre par les transducteurs du réseau d'imagerie, par itérations, les estimations précédemment obtenues des signaux de référence e'i(t,q), puis on capte avec les mêmes transducteurs des signaux $s_1(t,q)$ rétrodiffusés par le milieu hétérogène dissipatif, puis on modifie pour l'itération suivante ces signaux de référence e'i(t,q) de la manière suivante :

$$e'_i(t) \quad \rightarrow \quad \alpha_i(q) . e'_i(t - \tau_i(q))$$

où les valeurs $\alpha_1(q)$ et $\tau_i(q)$ sont un facteur d'amplitude et un retard correctifs, calculés pour maximiser un critère de cohérence C entre lesdits signaux rétrodiffusés, lesdites itérations étant arrêtées lorsque le critère C atteint un seuil prédéterminé,

(b3) on mémorise les signaux de référence e'i(t,q), au moins pour q compris entre m+1 et m+R,

(c) et une étape de focalisation au cours de laquelle, pour au moins un desdits points de focalisation q, on fait émettre respectivement par les transducteurs du réseau d'imagerie, lesdits signaux de référence e'$_1$(t,q), i étant un indice compris entre 1 et n désignant un transducteur du réseau d'imagerie.

Grâce à ces dispositions, on s'affranchit des aberrations de propagation des ondes acoustiques dans le milieu hétérogène dissipatif, et on obtient une focalisation très précise qui peut notamment permettre d'obtenir une image échographique fidèle et précise d'un champ à observer à travers la couche aberratrice par rétrodiffusion, lorsqu'on émet successivement des ondes acoustiques focalisées sur différents points du champ à observer et que l'on capte les ondes acoustiques rétrodiffusées.

Cette focalisation précise peut également être utilisée dans d'autres applications que l'échographie, notamment :

- l'imagerie doppler couleur,
- les méthodes d'imagerie par élastographie, telles que celle décrite dans le document WO-A-00/55 616,
- les méthodes d'imagerie non linéaire ("harmonic imaging"),
- les méthodes de traitement par destruction localisée d'une partie du milieu hétérogène dissipatif, notamment par hyperthermie,
- les méthodes de mesure de paramètres d'absorption optique des tissus avec activation par ultrasons, etc.

Dans des modes de réalisation préférés de l'invention, on peut éventuellement avoir recours en outre à l'une et/ou à l'autre des dispositions suivantes :

- au cours de la sous-étape (b11), lorsqu'au moins certains transducteurs (du réseau cible et/ou du réseau d'imagerie) sont en contact avec un milieu homogène intermédiaire (par exemple un gel) lui-même en contact avec la couche aberratrice, on corrige les réponses impulsionnelles hri(t) par repropagation numérique pour simuler des transducteurs situés directement au contact de la couche aberratrice ;
- la sous-étape (b12) comporte elle-même les sous-étapes suivantes :

  (b121) on détermine p matrices de transfert H(ωk)=[Hri(ωk)], i allant de 1 à n et r allant de 1 à m, où Hri(ωk) est la valeur, à la fréquence ωk, de la transformée de Fourier de la réponse impulsionnelle hri(t),
  (b122) on détermine pour chaque point de focalisation r correspondant à un transducteur du réseau cible, n composantes Ei(ωk,r), i variant entre 1 et n, telles que F(ωk,r)=H(ωk).E(ωk,r), où E(ωk,r) = [Ei(ωk,r)] est un vecteur à n composantes, F(ωk,r) est un vecteur à m composantes Fl(ωk,r), 1 variant entre 1 et m, ces m composantes Fl(ωk,r) correspondant à une focalisation souhaitée des ondes acoustiques à la fréquence ωk sur le point de focalisation r correspondant à un transducteur du réseau cible,
  (b123) on en déduit, pour chaque point de focalisation r correspondant à un transducteur du réseau cible, un vecteur de n signaux temporels e(t,r)=[ei(t,r)], i variant entre 1 et n, où $e_i(t,r) = \sum_{k=1}^{p} Ei(\omega k, r) . e^{j\omega k t}$ en notation

complexe, ces signaux ei(t,r) étant adaptés pour que l'émission de ces derniers respectivement par les différents transducteurs i du réseau d'imagerie génère une impulsion acoustique focalisée sur le point de focalisation r du réseau cible,

(b124) une sous-étape de correction des aberrations générées par la couche aberratrice entre le milieu sensiblement homogène et chaque transducteur cible r, ces aberrations étant estimées sur la base des mesures précédemment effectuées, les aberrations ainsi estimées étant utilisées pour calculer lesdits signaux temporels de référence e'i(t,r) ;

- au cours de la sous-étape (b122) on calcule p matrices $H^{-1}(\omega k)$, respectivement par régularisation et inversion des matrices de transfert $H(\omega k)$, et pour chaque transducteur r du réseau cible, on calcule le vecteur $E(\omega k,r)$ par la formule :

$$E(\omega k, r) = H^{-1}(\omega k) . F(\omega k, j) \quad ;$$

- au cours de l'étape (b122), les composantes $F1(\omega k,r)$ du vecteur $F(\omega k,r)$ correspondant à la répartition spatiale du champ désiré à la fréquence $\omega k$, sont égales à 0 pour $1 \neq r$ et égale à 1 pour $1=r$ ;

- au cours de la sous-étape (b124), on assimile la paroi aberratrice, au voisinage de chaque point de focalisation r correspondant à un transducteur du réseau cible, à un filtre à réponse impulsionnelle finie, défini à chaque fréquence $\omega k$ par une amplitude $Gr(\omega k)$ et une phase $\phi_r(\omega k)$, la sous-étape (b124) comportant elle-même les sous-étapes suivantes :

(b1241) on calcule, pour chaque fréquence $\omega k$, l'amplitude $Gr(\omega k)$ et la phase $\phi_r(\omega k)$, à partir soit des signaux ei(t,r), soit des vecteurs $E(\omega k, r)$,

(b1242) on calcule p matrices de transfert corrigées $H'(\omega k)=[H'ji(\omega k)]$, où

$$H'_{ji}(\omega_k) = H_{ji}(\omega_k) . \frac{1}{G_j(\omega_k)} e^{-j\phi_r(\omega_k)} ,$$

(b1243) on détermine pour chaque transducteur r du réseau cible, n composantes $E'i(\omega k,r)$, i variant entre 1 et n, telles que $F(\omega,r)=H'(\omega k).E'(\omega,r)$, où $E'(\omega k,r) = [Ei(\omega k,r)]$ est un vecteur à n composantes, $F(\omega k,r)$ est un vecteur à m composantes $Fl(\omega k,r)$, l variant entre 1 et m, ces m composantes $Fl(\omega k,r)$ correspondant à une focalisation souhaitée des ondes acoustiques à la fréquence $\omega k$ sur le point de focalisation r correspondant à un transducteur du réseau cible,

(b1244) on en déduit, pour chaque point de focalisation r correspondant à un transducteur du réseau cible, un vecteur de n signaux temporels de référence e'(t,r)=[e'i(t,r)], i variant entre 1 et n, où

$$e'_i(t,r) = \sum_{k=1}^{p} E'i(\omega k,r) . e^{j\omega k.t} \quad \text{en notation complexe ;}$$

- au cours de la sous-étape (b1241), on calcule l'amplitude $Gr(\omega k)$ et la phase $\phi_r(\omega k)$ comme suit :

$$G_r(\omega_k) = \frac{\sqrt{\sum_{i=1}^{n} Ei(\omega_k,r0).Ei^*(\omega_k,r0)}}{\sqrt{\sum_{i=1}^{n} Ei(\omega_k,r).Ei^*(\omega_k,r)}}$$

$$\phi_r(\omega_k) = \frac{1}{n}\sum_{i=1}^{n}\left(\arg(Ei(\omega_k,r0)) - \arg\left(Ei(\omega_k,r)e^{-j\Delta\tau(0,r,i)\omega k}\right)\right)$$

où :

- Ei$^*$ est la valeur complexe conjuguée de Ei,
- et $\Delta\tau(r0,r,i)=(d(r0,i)-d(r,i))/c$, $d(r,i)$ étant la distance entre le transducteur i et le point de focalisation r, et $d(r0,i)$ étant la distance entre le transducteur i et un point de focalisation particulier r0 ;

- la sous-étape (b12) comporte elle-même les sous-étapes suivantes :

   (b121) on détermine p matrices de transfert $H(\omega k)=[Hri(\omega k)]$, i allant de 1 à n et r allant de 1 à m, où $Hri(\omega k)$ est la valeur, à la fréquence $\omega k$, de la transformée de Fourier de la réponse impulsionnelle hri(t),
   (b122') on corrige les matrices de transfert $H(\omega k)$ pour s'affranchir des aberrations générées par la paroi aberratrice au voisinage de chaque point de focalisation r, cette correction étant effectuée à partir des réponses impulsionnelles hri(t) précédemment déterminées, et on obtient ainsi des matrices de transfert corrigées $H'(\omega k)$,
   (b123') on détermine pour chaque point de focalisation r correspondant à un transducteur du réseau cible, n composantes $E'i(\omega k,r)$, i variant entre 1 et n, telles que $F(\omega k,r)=H'(\omega k).E'(\omega k,r)$, où $E'(\omega k,r) = [E'i(\omega k,r)]$ est un vecteur à n composantes, $F(\omega k,r)$ est un vecteur à m composantes $Fl(\omega k,r)$, 1 variant entre 1 et m, ces m composantes $Fl(\omega k,r)$ correspondant à une focalisation souhaitée des ondes acoustiques à la fréquence $\omega k$ sur le point de focalisation r correspondant à un transducteur du réseau cible,
   (b124') on en déduit, pour chaque point de focalisation r correspondant à un transducteur du réseau cible, un vecteur de n signaux temporels $e'(t,r)=[e'i(t,r)]$, i variant entre 1 et n, où $e'_i(t,r)=\sum_{k=1}^{p} E'i(\omega k,r).e^{j\omega k.t}$ en

   notation complexe, les signaux $e'i(t,r)$ étant lesdits signaux de référence ;

- au cours de la sous-étape (b123') on calcule p matrices $H'^{-1}(\omega k)$, respectivement par régularisation et inversion des matrices de transfert $H'(\omega k)$, et pour chaque transducteur r du réseau cible, on calcule le vecteur $E'(\omega k,r)$ par la formule :

$$E'(\omega k,r)=H'^{-1}(\omega k).F(\omega k,j) \; ;$$

- au cours de l'étape (b123'), les composantes $Fl(\omega k,r)$ du vecteur $F(\omega k,r)$ correspondant à la répartition spatiale du champ désiré à la fréquence $\omega k$, sont égales à 0 pour $1 \neq r$ et égale à 1 pour $1=r$ ;
- au cours de la sous-étape (b122'), on assimile la paroi aberratrice, au voisinage de chaque point de focalisation r correspondant à un transducteur du réseau cible, à un filtre à réponse impulsionnelle finie, défini à chaque fréquence $\omega k$ par une amplitude $Gr(\omega k)$ et une phase $\phi_r(\omega k)$, la sous-étape (b122') comportant elle-même les sous-étapes suivantes :

   (b122'1) on calcule, pour chaque fréquence $\omega k$, l'amplitude $Gr(\omega k)$ et la phase $\phi_r(\omega k)$, à partir des réponses impulsionnelles précédemment déterminées,
   (b122'2) on calcule p matrices de transfert corrigées $H'(\omega k)=[H'ji(\omega k)]$, où

$$H'_{ji}(\omega_k) = H_{ji}(\omega_k).\frac{1}{G_j(\omega_k)}e^{-j\phi_{,(\omega_t)}} \; ;$$

- au cours de la sous-étape (b122'1) on calcule, pour chaque fréquence $\omega k$, l'amplitude $Gr(\omega k)$ et la phase $\phi_r(\omega k)$, de la manière suivante :

$$G_r(\omega_k)=\frac{\sqrt{\sum_{i=1}^{n}H_{ri}(\omega_k).H_{,i}^{\cdot}(\omega_k)}}{\sqrt{\sum_{i=1}^{n}H_{r0,i}(\omega_k).H_{r0,i}^{\cdot}(\omega_k)}}$$

$$\phi_r(\omega_k) = \frac{1}{n} \sum_{i=1}^{n} \left( \arg\left( H_{ri}(\omega_k) e^{j\Delta\tau(r,r,r_0)\omega_k} \right) - \arg\left( H_{r0,i}(\omega_k) \right) \right) ,$$

où :

- • H*ri désigne la valeur complexe conjuguée de Hri,
- • et $\Delta\tau(r0,r,i) = (d(r0,i) - d(r,i))/c$, d(r,i) étant la distance entre le transducteur i et le point de focalisation r, et d(r0,i) étant la distance entre le transducteur i et un point de focalisation particulier r0 ;

- au cours de l'étape (c), on fait suivre la sous-étape (c1) par les sous-étapes suivantes :

    (c2) on fait capter par lesdits transducteurs du réseau d'imagerie des signaux $s_i(t)$ rétrodiffusés par le milieu hétérogène dissipatif,
    (c3) on convolue le signal de référence émis par chaque transducteur du réseau d'imagerie avec le signal rétrodiffusé capté par ce transducteur,
    (c4) puis on somme les produits de convolution ainsi obtenus,

    l'étape (c) étant renouvelée pour une pluralité de points situés dans le milieu sensiblement homogène ;
- au cours de la sous-étape (b21), la première estimation de chaque signal de référence est $e'_i(t,q) = e'_i(ts+\theta i(q),q0)$ pour chaque point de focalisation q, q0 étant l'indice d'un point de focalisation proche du point q pour lequel le signal de référence a déjà été déterminé, $\theta i(q)$ étant un retard égal à une valeur $\delta i(q)/c$, où c est la célérité moyenne des ondes acoustiques dans le milieu, et $\delta i(q)$ est égal à une différence entre d'une part, une distance entre le transducteur i du réseau d'imagerie et le point de focalisation q0, et d'autre part, une distance entre le transducteur i du réseau d'imagerie et le point de focalisation q,
- au cours de la sous-étape (b2), lorsqu'au moins certains transducteurs d'indice v du réseau d'imagerie ne sont pas directement au contact de la couche aberratrice, on corrige les signaux $e'_v(t,q)$ correspondants par repropagation numérique pour simuler des transducteurs placés en contact direct avec la couche aberratrice ;
- au cours de la sous-étape (b22), on recherche les valeurs $\alpha_1(q)$ et $\tau_1(q)$ pour maximiser le critère de cohérence C suivant :

$$C = \frac{\left< \left| \sum_{i=1}^{n} \alpha_i \cdot g_i(i - \tau_i, q) \right|^2 \right>}{n \cdot \sum_{i=1}^{n} \left< \left| \alpha_i \cdot g_i(t - \tau_i, q) \right|^2 \right>} ,$$

où :

- • $g_1(t,q) = s_1(t) \otimes e_i(t,q)$, $\otimes$ représentant l'opération de convolution,
- • et <> représente une moyenne temporelle ;

- au cours de la sous-étape (b22), les valeurs $\tau_i(q)$ sont calculées en maximisant une fonction d'intercorrélation, pour des transducteurs voisins du réseau d'imagerie, des signaux $g_1(t,q)$ et $g_{i+1}(t,q)$ ;
- au cours de la sous-étape (b22), les valeurs $\alpha_i(q)$ sont calculées de manière à égaliser sur l'indice i l'amplitude maximale des fonctions $g_1(t,q)$ ;
- au cours de la sous-étape (b22), les valeurs $\alpha_i(q)$ et $\tau_i(q)$ sont calculées en réalisant une intercorrélation, pour des transducteurs voisins du réseau d'imagerie, des signaux $g_1(t,q)$ et $g_{i+1}(t,q)$ ;
- au cours de la sous-étape (b22), les valeurs $\alpha_1(q)$ et $\tau_i(q)$ sont calculées de manière à égaliser sur l'indice i l'amplitude maximale des fonctions $g_i(t,q)$ ;
- la sous-étape (b22) relative à chaque point de focalisation q est réalisée immédiatement après la sous-étape (b21) relative au même point de focalisation q ;
- le milieu hétérogène dissipatif est constitué par le cerveau entouré par le crâne ;

- le réseau d'imagerie et le réseau cible sont deux réseaux distincts disposés de part et d'autre du milieu hétérogène dissipatif ;
- tous les transducteurs appartiennent à la fois au réseau d'imagerie et au réseau cible ;
- les ondes acoustiques sont des ondes ultrasonores.

Par ailleurs, l'invention a également pour objet un dispositif adapté pour mettre en oeuvre le procédé défini ci-dessus.

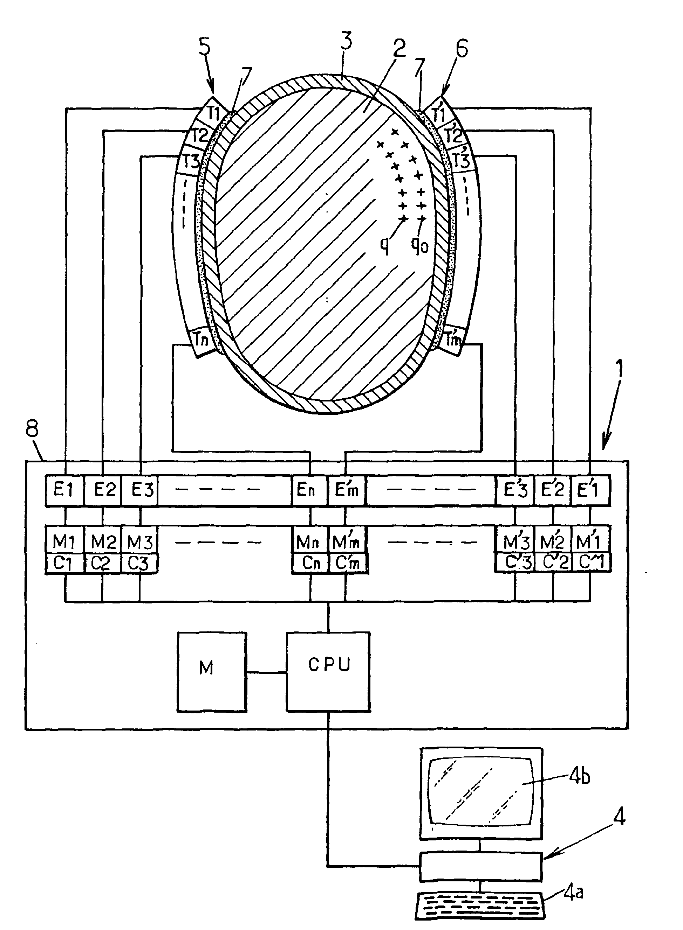

D'autres caractéristiques et avantages de l'invention apparaîtront au cours de la description suivante d'une de ses formes de réalisation, donnée à titre d'exemple non limitatif, en regard du dessin joint.

Sur le dessin, la figure unique représente un dispositif d'imagerie ultrasonore selon une forme de réalisation de l'invention.

Le dispositif 1 d'imagerie ultrasonore représenté sur le dessin est adapté pour réaliser une image échographique par ultrasons du cerveau 2 d'un patient (à des fréquences par exemple de l'ordre de 1 à 3 MHz), depuis l'extérieur du crâne 3, le cerveau 2 constituant un milieu sensiblement homogène pour la propagation des ondes acoustiques et le crâne 3 constituant une couche aberratrice dissipative, de sorte que l'ensemble de la boîte crânienne 2, 3 constitue un milieu hétérogène dissipatif.

En variante, l'invention serait applicable notamment :

- à l'imagerie acoustique de tout autre milieu hétérogène dissipatif non homogène comprenant un milieu sensiblement homogène entouré par une couche dissipative relativement peu épaisse générant des aberrations dans la propagation des ondes ultrasonores,
- ou à tout autre procédé impliquant au moins une focalisation à l'émission dans un tel milieu.

Dans l'exemple représenté sur le dessin, le dispositif d'imagerie 1 comporte un micro-ordinateur 4, ou tout autre dispositif de commande et/ou de visualisation des images ultrasonores, ce micro-ordinateur comportant classiquement un clavier 4a associé éventuellement à d'autres interfaces de commande et un écran 4b permettant de visualiser les images du cerveau 2.

Par ailleurs, le dispositif d'imagerie 1 comporte deux réseaux 5, 6 de transducteurs ultrasons T1, T2... Tn et T'1, T'2... T'm formant par exemple deux barrettes linéaires de transducteurs qui sont disposées de part et d'autre du crâne 3 de l'utilisateur, dans des positions géométriques prédéterminées l'une par rapport à l'autre, chaque barrette de transducteur 5, 6 étant mise en contact avec le crâne 3 par l'intermédiaire d'une couche 7 de gel ou similaire.

Les différents transducteurs T1, T2... Tn et T'1, T'2... T'm peuvent être commandés directement par le micro-ordinateur 4, ou de préférence par une unité centrale électronique CPU contenue par exemple dans une baie électronique 8 et elle-même commandée par le micro-ordinateur 4.

Avantageusement, chacun des transducteurs T1, T2... Tn, T'1, T'2, T'm est relié à un échantillonneur, respectivement E1, E2... En, E'1, E'2, E'm, et chaque échantillonneur est lui-même relié à une mémoire, respectivement M1, M2... Mm, M'1, M'2... M'm et à une unité centrale C1, C2, ...Cm,C'1,C'2, ... C'm. Ces mémoires et ces unités centrales sont à leur tour reliés, directement ou indirectement, à l'unité centrale CPU susmentionnée, qui est par ailleurs reliée au moins à une mémoire centrale M.

Le dispositif qui vient d'être décrit fonctionne comme suit.

Initialement, les deux réseaux de transducteurs 5, 6 sont fixés de part et d'autre du crâne 3 du patient, dans lesdites positions prédéterminées. A cet effet, les réseaux de transducteurs 5, 6, dits respectivement réseau d'imagerie et réseau cible, peuvent être portés par un support rigide tel qu'un casque (non représenté) disposé autour de la tête du patient.

Ensuite, le dispositif suit une étape d'apprentissage de quelques minutes (avantageusement 1 à 3 minutes) permettant de prendre en compte l'ensemble des aberrations de propagation dues au caractère non homogène du milieu dissipatif formé par le crâne 3 et le cerveau 2.

Au cours de cette étape d'apprentissage on fait d'abord émettre successivement par chacun des transducteurs T1, T2... Ti,... Tn du réseau d'imagerie 5, une impulsion acoustique, et pour chaque impulsion émise par l'un des transducteurs Ti du réseau d'imagerie, on enregistre le signal capté par les transducteurs T'1, T'1... T'r,... T'm du réseau cible 6, c'est-à-dire la réponse impulsionnelle $hri(t)$ du milieu hétérogène dissipatif entre le transducteur i considéré du réseau d'imagerie 5 et chaque transducteur j du réseau cible 6.

Chaque réponse impulsionnelle $hri(t)$ est enregistrée sous forme numérique avec un certain échantillonnage temporel qui détermine un certain nombre p de composantes fréquentielles monochromatiques de la réponse impulsionnelle, correspondant chacune à une fréquence $\omega k$, k étant un indice compris entre 1 et p.

Dans le cas envisagé ici, où au moins certains transducteurs du réseau cible et/ou du réseau d'imagerie ne sont pas directement au contact avec la couche aberratrice 3, on corrige les réponses impulsionnelles pour simuler des transducteurs virtuels disposés au contact de ladite couche aberratrice. La position de la couche par rapport aux transducteurs peut éventuellement être obtenue par imagerie conventionnelle (échographie ultrasonore, scanner X, IRM, etc.). Les réponses impulsionnelles corrigées sont calculées par un algorithme de repropagation numérique connu, décrit notamment dans les articles suivants :

- "Ultrasonic beam steering through inhomogeneous layers with a time reversal mirror", C.DORME, M. FINK, IEEE Transactions Ultrasonics, Ferroelectric and Frequency Control, 43 (1), janvier 1996,p 167-175,
- "Focusing and steering through absorbing and aberrating layers : Application to ultrasonic propagation through the skull" Journal of Acoustical Society of America, 103 (5), Mai 1998, p. 2403-2410,
- et "Propagation and backpropagation for ultrasonic wavefront design" Liu, D.-L., and Waag, R. C. IEEE Trans. on Ultras. Ferro. and Freq. Contr. 44(1):1-13 (1997).

Dans ce qui suit, hri(t) dénommera donc les réponses impulsionnelles pour des éléments (réels ou virtuels) situés contre la couche aberratrice. De plus, les éléments virtuels ou réels situés contre la couche aberratrice 3 seront appelés ci-après "points de focalisation" d'indice r compris entre 1 et m.

Lorsque les transducteurs du réseau d'imagerie 5 émettent des signaux acoustiques $e_1(t)$, ces signaux génèrent au niveau des transducteurs r du réseau cible 6 des signaux acoustiques fr(t) s'exprimant comme suit :

$$fr(t) = \sum_{i=1}^{n} \mathrm{hri(t)} \otimes \mathrm{ei(t)},$$

où $\otimes$ représente l'opérateur de convolution temporelle.

Après transformée de Fourier, cette équation devient :

$$F(\omega k) = H(\omega k) . E(\omega k),$$

où :

- $H(\omega k)$ est la matrice de transfert, de taille m*n, entre les transducteurs Ti du réseau d'imagerie et les transducteurs Tr du réseau cible : les composantes $Hri(\omega k)$ de cette matrice sont les composantes des transformées de Fourier des réponses impulsionnelles hri(t) à la fréquence $\omega k$,
- $E(\omega k)$ est un vecteur dont les composantes $E_1(\omega k)$ sont les composantes de la transformée de Fourier des signaux $e_1(t)$ susmentionnés à la fréquence $\omega k$,
- et $F(\omega k)$ est un vecteur dont les composantes $F_J(\omega k)$ sont les composantes de la transformée de Fourrier des signaux $f_J(t)$ susmentionnés à la fréquence $\omega k$.

Par inversion de chaque matrice de transfert $H(\omega k)$, on peut donc déterminer le vecteur $E(\omega k,j)$ qui est adapté pour générer au niveau du point de focalisation r correspondant au transducteur T'r du réseau cible, un vecteur $F(\omega k, j)$ dont toutes les composantes se rapprochent au mieux de l'objectif initialement fixé (de préférence toutes égales à zéro, sauf la composante d'indice j correspondant au transducteur T'j, qui est égale à 1 lorsqu'on veut émettre une impulsion acoustique au niveau du point de focalisation r), grâce à la relation :

$$E(\omega k, j) = H^{-1}(\omega k) . F(\omega k, j),$$

où $H^{-1}(\omega k)$ est la matrice inverse de $H(\omega k)$.

$H^{-1}(\omega k)$ peut être calculée par exemple par décomposition en valeurs singulières, ceci permettant une régularisation de l'inversion de la matrice $H(\omega k)$.

Puis on détermine, par transformée de Fourier inverse des différentes composantes $Ei(\omega k,j)$ du vecteur $E(\omega k, j)$, les différents signaux de référence ei(t,j) qui, lorsqu'ils sont émis par les différents transducteurs Ti du réseau d'imagerie 5, sont adaptés pour focaliser une impulsion acoustique (ou le cas échéant un autre signal acoustique) au niveau du point de focalisation r. On a donc réalisé une focalisation du réseau d'imagerie 5 sur chaque transducteur du réseau cible 6, par filtre inverse spatio-temporel.

L'unité centrale CPU suit ensuite un processus d'apprentissage des aberrations dues à la paroi du crâne 3 au niveau du réseau cible.

Au cours de ce processus, ces aberrations sont considérées comme un filtre à réponse impulsionnelle finie.

Dans le domaine de Fourier, ce filtre est défini à chaque fréquence ωk une amplitude Gr(ωk) et une phase $\phi_r$(ωk).

Pour calculer ces coefficients, on compare la phase et l'amplitude de l'ensemble des vecteurs Er. A cet effet, on commence par éliminer les déphasages introduits par les différences de marche entre les transducteurs d'imagerie Ti et les différents points de focalisation indicés r. Ceci revient à choisir un point de focalisation particulier r0, et introduire pour les autres un déphasage linéaire avec la pulsation : exp(-jΔτ(r0,r,i)ω) avec Δτ(r0,r,i)=(d(r0,i)-d(r,i))/c où d(r,i) est la distance entre le transducteur i et le point de focalisation r et c est la vitesse moyenne des ondes acoustiques dans le milieu à imager, en l'occurrence le cerveau 2.

Cette correction effectuée, les différences d'amplitude et de phase entre les vecteurs Er sont attribuées à la couche aberratrice 3 situées contre le réseau cible. On calcule alors pour chaque point de focalisation r, le facteur de gain Gj(ωk) et le facteur de phase $\phi_j$(ωk) :

$$G_r(\omega_k)=\frac{\sqrt{\sum_{i=1}^{n}E_i(\omega_k,r0).E_i^*(\omega_k,r0)}}{\sqrt{\sum_{i=1}^{n}E_i(\omega_k,r).E_i^*(\omega_k,r)}}$$

$$\phi_r(\omega_k)=\frac{1}{n}\sum_{i=1}^{n}\left(\arg(E_i(\omega_k,r0))-\arg\left(E_i(\omega_k,r)e^{-j\Delta\tau(r0,r,t)\omega_k}\right)\right)$$

où Ei* est la valeur complexe conjuguée de Ei.

Ces couples {Gj(ωk),$\phi_J$(ωk)} correspondent au facteur d'atténuation et au déphasage relatifs introduits à chaque fréquence par la portion de couche aberratrice 3 située contre le point de focalisation r. Ils caractérisent donc finalement les aberrations introduites par la portion de couche aberratrice située contre le réseau cible.

On élimine ensuite les aberrations introduites par la couche aberratrice 3 située contre les transducteurs cibles, dans l'ensemble des p matrices H(ωk)=[Hji(ωk)], définies ci-dessus.

Pour cela, on calcule un nouvel ensemble de matrices de transfert H'(ωk)=[H'ji(ωk)] caractérisant la propagation entre le réseau d'imagerie et le réseau cible dans un milieu virtuel pour lequel seules subsistent les aberrations situées contre le réseau d'imagerie :

$$H'_{ji}(\omega_k) = H_{ji}(\omega_k).\frac{1}{G_j(\omega_k)}e^{-j\phi_i(\omega_k)}.$$

On détermine alors pour chaque transducteur r du réseau cible, n composantes E'i(ωk,r), i variant entre 1 et n, telles que F(ωk,r)=H'(ωk).E'(ωk,r), où E'(ωk,r) = [E'i(ωk,r)] est un vecteur à n composantes, F(ωk,r) est un vecteur à m composantes Fl(ωk,r), 1 variant entre 1 et m, ces m composantes Fl(ωk,r) correspondant à une focalisation souhaitée des ondes acoustiques à la fréquence ωk sur le point de focalisation r correspondant à un transducteur du réseau cible.

On en déduit, pour chaque point de focalisation r correspondant à un transducteur du réseau cible, un vecteur de n signaux temporels de référence e'(t,r)=[e'i(t,r)], i variant entre 1 et n, où $e'_i(t,r)=\sum_{k=1}^{p}E'_i(\omega_k,r).e^{j\omega_k.t}$ en notation complexe.

Ces signaux de référence e'i(t,j) sont adaptés pour que l'émission de ces derniers respectivement par les différents transducteurs i du réseau d'imagerie génère une impulsion acoustique focalisée sur le transducteur j du réseau cible en l'absence de la couche aberratrice située contre le réseau cible.

On notera qu'en variante, les signaux de référence pourraient être déterminés de la façon suivante après la détermination des réponses impulsionnelles hri(t) et des p matrices de transfert H(ωk) :

- on corrige les matrices de transfert H(ωk) pour s'affranchir des aberrations générées par la paroi aberratrice 3 au voisinage de chaque point de focalisation r, cette correction étant effectuée à partir des réponses impulsionnelles hri(t) précédemment déterminées, et on obtient ainsi des matrices de transfert corrigées H'(ωk),

- on détermine par inversion des matrices H'(ωk), pour chaque point de focalisation r correspondant à un transducteur du réseau cible, n composantes E'i(ωk,r), i variant entre 1 et n, telles que F(ωk,r)=H'(ωk).E'i(ωk,r), où E'i(ωk,r) = [E'i(ωk,r)] est un vecteur à n composantes, F(ωk,r) est un vecteur à m composantes Fl(ωk,r), 1 variant entre 1 et m, ces m composantes Fl(ωk,r) correspondant à une focalisation souhaitée des ondes acoustiques à la fréquence ωk sur le point de focalisation r correspondant à un transducteur du réseau cible,

- et on en déduit, pour chaque point de focalisation r correspondant à un transducteur du réseau cible, un vecteur de n signaux temporels e'(t,r)= [e'i(t,r)], i variant entre 1 et n, où $e'_i(t,r) = \sum_{k=1}^{p} E'i(\omega k, r) . e^{j\omega k . t}$ en notation complexe, les signaux e'i(t,r) étant lesdits signaux de référence.

Avantageusement, lors du calcul des matrices H'(ωk), on assimile la paroi aberratrice, au voisinage de chaque point de focalisation r correspondant à un transducteur du réseau cible, à un filtre à réponse impulsionnelle finie, défini à chaque fréquence ωk par une amplitude Gr(ωk) et une phase $\phi_r(\omega k)$ calculés comme suit :

$$G_r(\omega_k) = \frac{\sqrt{\sum_{i=1}^{n} H_{ri}(\omega_k) . H_{ri}^{\cdot}(\omega_k)}}{\sqrt{\sum_{i=1}^{n} H_{r0,i}(\omega_k) . H_{r0,i}^{\cdot}(\omega_k)}}$$

$$\phi_r(\omega_k) = \frac{1}{n} \sum_{i=1}^{n} \left( \arg\left( H_{ri}(\omega_k) e^{j\Delta\tau(r,r0)\omega k} \right) - \arg\left( H_{r0,i}(\omega_k) \right) \right) ,$$

où :

- H*ri désigne la valeur complexe conjuguée de Hri,
- et Δτ(r0,r,i)=(d(r0,i)-d(r,i))/c, d(r,i) étant la distance entre le transducteur i et le point de focalisation r, et d(r0,i) étant la distance entre le transducteur i et un point de focalisation particulier r0.

On calcule ensuite p matrices de transfert corrigées H'(ωk)=[H'ji(ωk)], où

$$H_{ji}^{'}(\omega_k) = H_{ji}^{\cdot}(\omega_k) . \frac{1}{G_j(\omega_k)} e^{-j\phi_j(\omega_k)} ,$$ qui servent à déterminer les vecteurs E'i(ωk) comme explicité précédemment et donc les différents signaux de référence e'i(t,r), r allant de 1 à m.

L'unité centrale CPU procède ensuite à un apprentissage de focalisation en un nombre R de points de focalisation prédéterminés situés dans le cerveau 2, d'indices q compris entre m+1 et m+R, cette sous-étape consistant à déterminer pour chacun de ces points de focalisation q, en s'éloignant pas à pas des transducteurs du réseau cible, des signaux de référence e'i(t,q) à faire émettre par les différents transducteurs du réseau d'imagerie pour générer une impulsion focalisée sur ledit point de focalisation q.

Les signaux de référence e'i(t,q) sont initialement déterminés, pour chaque nouveau point de focalisation q, sous la forme e'i(t,q) = e'i(t+θi(q), q0) pour chaque point de focalisation q, q0 étant l'indice d'un point de focalisation proche du point q pour lequel le signal de référence a déjà été déterminé, le retard θi(q) étant initialement égal à une valeur δi(q)/c, où c est la valeur moyenne de célérité des ondes acoustiques dans le milieu, et δi(q) est égal à une différence entre d'une part, une distance entre le transducteur i du réseau d'imagerie et le point de focalisation q0, et d'autre part, une distance entre le transducteur i du réseau d'imagerie et le point de focalisation q.

Dans le cas où certains transducteurs d'indice v du réseau d'imagerie ne sont pas situés contre la couche aberratrice, il est en outre souhaitable de corriger les signaux de référence les signaux e'v(t,q) correspondants par repropagation numérique des transducteurs virtuels (situés contre la couche aberratrice 3) aux transducteurs réels (séparés de ladite couche 3 par du gel 7 ou autre), de façon connue en soi, par le procédé inverse de celui décrit ci-dessus en ce qui concerne les réponses impulsionnelles.

On fait ensuite émettre par les transducteurs du réseau d'imagerie, par itérations, les estimations obtenues des signaux de référence e'i(t,q), puis on capte avec les mêmes transducteurs les signaux, $s_i(t,q)$, rétrodiffusés par le milieu hétérogène dissipatif.

Puis, on modifie pour l'itération suivante ces signaux de référence e'i(t,q) de la manière suivante :

$$ e_i^{'}(t) \quad \rightarrow \quad \alpha_i(q) \cdot e_i^{'}(t - \tau_i(q)) $$

où les valeurs $\tau_i(q)$ et $\alpha_1(q)$ sont un retard et un facteur d'amplitude correctifs, calculées pour maximiser un critère de cohérence C entre lesdits signaux rétrodiffusés.

Avantageusement, le critère C pourra être le suivant :

$$ C = \frac{< \left| \sum_{i=1}^{n} \alpha_i \cdot g_i(t - \tau_i, q) \right|^2 >}{n \cdot \sum_{i=1}^{n} < \left| \alpha_i \cdot g_i(t - \tau_i, q) \right|^2 >} \quad , $$

où :

- $g_1(t,q) = s_i(t) \otimes e_1(t,q)$, $\otimes$ représentant l'opération de convolution,
- et <> représente une moyenne temporelle.

Dans ce processus d'optimisation, les valeurs $\tau_1(q)$ peuvent être calculées à chaque itération de façon à maximiser une fonction d'intercorrélation, pour des transducteurs voisins du réseau d'imagerie, des signaux $g_1(t,q)$ et $g_{i+1}(t,q)$ susmentionnés, et les valeurs $\alpha_i(q)$ peuvent être calculées de manière à égaliser sur l'indice i l'amplitude maximale des fonctions $g_1(t,q)$.

On optimise ainsi les signaux de référence e'i(t,q), i allant de 1 à n, de façon qu'ils produisent un signal acoustique focalisé de façon précise sur le point de focalisation q situé dans le cerveau. Ce processus d'optimisation a déjà été explicité plus en détail par Mallart et al. (The Van Cittert-Zernike theorem in pulse echo measurements, J. Acoust. Soc. Am. 90(5), novembre 1991, p. 2716-2727 ; Adaptative focusing in scattering media through sound speed inhomogeneities : the Van Cittert Zernike approach and focusing criterion, J. Acoust. Soc. Am. 96(6), décembre 1994, p. 3721-3732).

Lorsque cette optimisation est terminée pour un point de focalisation q, par exemple après 2 ou 3 itérations lorsque le critère C a atteint une valeur prédéterminée (notamment proche de 2/3), on passe au point de focalisation q+1 suivant, etc.

Les signaux de référence e'i(t,q) ainsi obtenus sont mémorisés, par exemple dans les mémoires M1-Mn.

Une fois l'étape d'apprentissage terminée, on peut notamment réaliser des images échographiques du cerveau 2, éventuellement à un rythme élevé pouvant atteindre la cadence d'un échographe standard, par exemple 20 à 30 images par seconde. Pour réaliser chacune de ces images, on procède comme suit, pour chaque point de focalisation q appartenant au champ à observer :

- on fait émettre respectivement par les transducteurs Ti du réseau d'imagerie, lesdits signaux de référence ei(t,q),
- puis on fait capter par lesdits transducteurs du réseau d'imagerie des signaux si(t) rétrodiffusés par le milieu viscoélastique,
- on convolue le signal de référence ei(t,q) émis par chaque transducteur du réseau d'imagerie avec le signal rétrodiffusé si(t) capté par ce transducteur,
- puis on somme les produits de convolution ainsi obtenus.

On notera que les différentes opérations susmentionnées réalisées au cours de l'étape d'apprentissage ou de l'étape d'imagerie peuvent être soit programmées dans l'unité centrale CPU, soit réalisées en tout ou partie par des circuits spécialisés.

Par ailleurs, on notera également que tous les transducteurs Ti, T'r pourraient être utilisés pour réaliser les images échographiques du cerveau. Dans ce cas, le réseau d'imagerie serait confondu avec le réseau cible et chacun de ces deux réseaux comprendrait tous les transducteurs, le fonctionnement décrit ci-dessus s'appliquant alors mutatis mutandis.

**Revendications**

**1.** Procédé non invasif de focalisation d'ondes acoustiques dans un milieu hétérogène dissipatif (2,3) comprenant un milieu sensiblement homogène (2) entouré au moins partiellement par une couche aberratrice dissipative (3) qui génère des aberrations dans la propagation des ondes acoustiques, les ondes acoustiques étant émises depuis l'extérieur de la couche aberratrice (3) et focalisées dans le milieu sensiblement homogène (2), comportant les étapes suivantes :

(a) une étape initiale de positionnement au cours de laquelle on fixe un nombre t supérieur à 2 de transducteurs acoustiques (T1-Tn, T'1-T'm) dans des positions prédéterminées à l'extérieur de la couche aberratrice (3), ces transducteurs étant en contact avec ladite couche aberratrice et formant au moins :

- un réseau d'imagerie (T1-Tn) qui regroupe un nombre n compris entre 1 et t desdits transducteurs,
- et un réseau cible (T'1-T'm) qui regroupe un nombre m compris entre 1 et t desdits transducteurs,

(b) une étape d'apprentissage comprenant elle-même les sous étapes suivantes :

(b1) une sous-étape d'apprentissage de focalisation du réseau d'imagerie sur le réseau cible, sous-étape au cours de laquelle :

(b11) on détermine des réponses impulsionnelles hri(t) du milieu hétérogène dissipatif, respectivement entre chaque transducteur i du réseau d'imagerie et plusieurs points de focalisation r situés sur la couche aberratrice (3) en correspondance respective avec des transducteurs du réseau cible, ces réponses impulsionnelles étant mémorisées sous forme numérique avec un certain échantillonnage temporel qui détermine un nombre p de composantes fréquentielles de la réponse impulsionnelle, de fréquences respectives $\omega k$, i étant un indice compris entre 1 et n qui désigne un transducteur du réseau d'imagerie, r étant un indice compris entre 1 et m qui désigne un point de focalisation correspondant à un transducteur du réseau cible et k étant un indice compris entre 1 et p qui désigne une composante fréquentielle,

(b12) à partir de ces réponses impulsionnelles, on calcule, pour chaque point de focalisation r correspondant à un transducteur du réseau cible, un ensemble de n signaux de référence temporels e'i(t,r)., i variant entre 1 et n, tels que, si la paroi aberratrice était enlevée au voisinage du point de focalisation r, l'émission de ces signaux de référence par les différents transducteurs i du réseau d'imagerie génèrerait un signal prédéterminé focalisé sur le point de focalisation r,

(b2) une sous-étape de focalisation en un nombre R de points de focalisation prédéterminés situés dans le milieu sensiblement homogène, d'indices q compris entre m+1 et m+R, cette sous-étape consistant à déterminer pour chacun de ces points de focalisation q, en s'éloignant pas à pas des points de focalisation 1 à m correspondant aux transducteurs du réseau cible, des signaux de référence e'i(t,q) à faire émettre par les différents transducteurs i du réseau d'imagerie pour générer une impulsion focalisée sur ledit point de focalisation q, les signaux de référence e'i(t,q) étant déterminés pour chaque point de focalisation q en procédant comme suit :

(b21) une première estimation de e'i(t,q), pour i allant de 1 à n, est calculée à partir d'au moins un signal de référence e'i(t,q0), q0 étant l'indice d'au moins un point de focalisation proche du point de focalisation q pour lequel le signal de référence a déjà été déterminé, ce calcul étant fait en utilisant une célérité moyenne des ondes acoustiques dans le milieu sensiblement homogène (2),

(b22) on fait émettre par les transducteurs du réseau d'imagerie, par itérations, les estimations précédemment obtenues des signaux de référence e'i(t,q), puis on capte avec les mêmes transducteurs des signaux $s_1(t,q)$ rétrodiffusés par le milieu hétérogène dissipatif, puis on modifie pour l'itération suivante ces signaux de référence e'i(t,q) de la manière suivante :

$$e'_i(t) \;\rightarrow\; \alpha_i(q) \cdot e'_i(t - \tau_i(q))$$

où les valeurs $\alpha_i(q)$ et $\tau_i(q)$ sont un facteur d'amplitude et un retard correctifs, calculés pour maximiser un critère de cohérence C entre lesdits signaux rétrodiffusés, lesdites itérations étant arrêtées lorsque le critère C atteint un seuil prédéterminé,

(b3) on mémorise les signaux de référence e'i(t,q), au moins pour q compris entre m+1 et m+R,

(c) et une étape de focalisation au cours de laquelle, pour au moins un desdits points de focalisation q, on fait émettre respectivement par les transducteurs du réseau d'imagerie, lesdits signaux de référence e'i(t,q), i étant un indice compris entre 1 et n désignant un transducteur du réseau d'imagerie.

**2.** Procédé selon la revendication 1, dans lequel au cours de la sous-étape (b11), lorsqu'au moins certains transducteurs (T1-Tm, T'1-T'm) sont en contact avec un milieu homogène intermédiaire lui-même en contact avec la couche aberratrice, on corrige les réponses impulsionnelles hri(t) par repropagation numérique pour simuler des transducteurs situés directement au contact de la couche aberratrice.

**3.** Procédé selon la revendication 1 ou la revendication 2, dans lequel la sous-étape (b12) comporte elle-même les sous-étapes suivantes :

(b121) on détermine p matrices de transfert H($\omega$k)=[Hri($\omega$k)], i allant de 1 à n et r allant de 1 à m, où Hri($\omega$k) est la valeur, à la fréquence $\omega$k, de la transformée de Fourier de la réponse impulsionnelle hri(t),

(b122) on détermine pour chaque point de focalisation r correspondant à un transducteur du réseau cible, n composantes Ei($\omega$k,r), i variant entre 1 et n, telles que F($\omega$k,r)=H($\omega$k).E($\omega$k,r), où E($\omega$k,r) = [Ei($\omega$k,r)] est un vecteur à n composantes, F($\omega$k,r) est un vecteur à m composantes Fl($\omega$k,r), l variant entre 1 et m, ces m composantes Fl($\omega$k,r) correspondant à une focalisation souhaitée des ondes acoustiques à la fréquence $\omega$k sur le point de focalisation r correspondant à un transducteur du réseau cible,

(b123) on en déduit, pour chaque point de focalisation r correspondant à un transducteur du réseau cible, un vecteur de n signaux temporels e(t,r)=[ei(t,r)], i variant entre 1 et n, où $e_i(t,r)=\sum_{k=1}^{p}Ei(\omega k,r).e^{j\omega k.t}$ en notation complexe, ces signaux ei(t,r) étant adaptés pour que l'émission de ces derniers respectivement par les différents transducteurs i du réseau d'imagerie génère une impulsion acoustique focalisée sur le point de focalisation r du réseau cible,

(b124) une sous-étape de correction des aberrations générées par la couche aberratrice entre le milieu sensiblement homogène et chaque transducteur cible r, ces aberrations étant estimées sur la base des mesures précédemment effectuées, les aberrations ainsi estimées étant utilisées pour calculer lesdits signaux temporels de référence e'i(t,r).

**4.** Procédé selon la revendication 3, dans lequel au cours de la sous-étape (b122) on calcule p matrices H$^{-1}$($\omega$k), respectivement par régularisation et inversion des matrices de transfert H($\omega$k), et pour chaque transducteur r du réseau cible, on calcule le vecteur E($\omega$k,r) par la formule :

$$E(\omega k, r) = H^{-1}(\omega k) . F(\omega k, j).$$

**5.** Procédé selon la revendication 3 ou la revendication 4, dans lequel au cours de l'étape (b122), les composantes Fl($\omega$k,r) du vecteur F($\omega$k,r) correspondant à la répartition spatiale du champ désiré à la fréquence $\omega$k, sont égales à 0 pour l≠r et égale à 1 pour l=r.

**6.** Procédé selon l'une quelconque des revendications 3 à 5, dans lequel au cours de la sous-étape (b124), on assimile la paroi aberratrice, au voisinage de chaque point de focalisation r correspondant à un transducteur du réseau cible, à un filtre à réponse impulsionnelle finie, défini à chaque fréquence $\omega$k par une amplitude Gr($\omega$k) et une phase $\phi_r$($\omega$k), la sous-étape (b124) comportant elle-même les sous-étapes suivantes :

(b1241) on calcule, pour chaque fréquence $\omega$k, l'amplitude Gr($\omega$k) et la phase $\phi_r$($\omega$k), à partir soit des signaux ei(t,r), soit des vecteurs E($\omega$k, r),

(b1242) on calcule p matrices de transfert corrigées H'($\omega$)=[H'ji($\omega$k)], où

$$H'_{ji}(\omega_k) = H_{ji}(\omega_k) \cdot \frac{1}{G_j(\omega_k)} e^{-j\phi_r(\omega_k)} \ ,$$

(b1243) on détermine pour chaque transducteur r du réseau cible, n composantes E'i($\omega$k,r), i variant entre 1 et n, telles que F($\omega$k,r)=H'($\omega$k).E'($\omega$k,r), où E'($\omega$k,r)= [E'i($\omega$k,r)] est un vecteur à n composantes, F($\omega$k,r) est un vecteur à m composantes Fl($\omega$k,r), 1 variant entre 1 et m, ces m composantes Fl($\omega$k,r) correspondant à une focalisation souhaitée des ondes acoustiques à la fréquence $\omega$k sur le point de focalisation r correspondant à un transducteur du réseau cible,

(b1244) on en déduit, pour chaque point de focalisation r correspondant à un transducteur du réseau cible, un vecteur de n signaux temporels de référence e'(t,r)=[e'i(t,r)], i variant entre 1 et n, où

$$e'_i(t,r) = \sum_{k=1}^{p} E'i(\omega k,r) \cdot e^{j\omega k\,t} \quad \text{en notation complexe.}$$

**7.** Procédé selon la revendication 6, dans lequel au cours de la sous-étape (b1241), on calcule l'amplitude Gr($\omega$k) et la phase $\phi_r$($\omega$k) comme suit :

$$G_r(\omega k) = \frac{\sqrt{\sum_{i=1}^{n} Ei(\omega k, r0) \cdot Ei^*(\omega k, r0)}}{\sqrt{\sum_{i=1}^{n} Ei(\omega k, r) \cdot Ei^*(\omega k, r)}}$$

$$\phi_r(\omega k) = \frac{1}{n}\sum_{i=1}^{n}\left(\arg\big(Ei(\omega k, r0)\big) - \arg\big(Ei(\omega k, r) e^{-j\Delta\tau(r0,r,i)\omega k}\big)\right)$$

où :

. Ei$^*$ est la valeur complexe conjuguée de Ei,
. et $\Delta\tau$(r0,r,i)=(d(r0,i)-d(r,i))/c, d(r,i) étant la distance entre le transducteur i et le point de focalisation r, et d(r0,i) étant la distance entre le transducteur i et un point de focalisation particulier r0.

**8.** Procédé selon la revendication 1 ou la revendication 2, dans lequel la sous-étape (b12) comporte elle-même les sous-étapes suivantes :

(b121) on détermine p matrices de transfert H($\omega$k)=[Hri($\omega$k)], i allant de 1 à n et r allant de 1 à m, où Hri($\omega$k) est la valeur, à la fréquence $\omega$k, de la transformée de Fourier de la réponse impulsionnelle hri(t),

(b122') on corrige les matrices de transfert H($\omega$k) pour s'affranchir des aberrations générées par la paroi aberratrice au voisinage de chaque point de focalisation r, cette correction étant effectuée à partir des réponses impulsionnelles hri(t) précédemment déterminées, et on obtient ainsi des matrices de transfert corrigées H'($\omega$k),

(b123') on détermine pour chaque point de focalisation r correspondant à un transducteur du réseau cible, n composantes E'i($\omega$k,r), i variant entre 1 et n, telles que F($\omega$k,r)=H' ($\omega$k).E'($\omega$k,r), où E'($\omega$k,r)= [E'i($\omega$k,r)] est un vecteur à n composantes, F($\omega$k,r) est un vecteur à m composantes Fl($\omega$k,r), l variant entre 1 et m, ces m composantes Fl($\omega$k,r) correspondant à une focalisation souhaitée des ondes acoustiques à la fréquence $\omega$k sur le point de focalisation r correspondant à un transducteur du réseau cible,

(b124') on en déduit, pour chaque point de focalisation r correspondant à un transducteur du réseau cible, un vecteur de n signaux temporels e'(t,r)=[e'i(t,r)], i variant entre 1 et n, où $e'_i(t,r) = \sum_{k=1}^{p} E'i(\omega k,r) \cdot e^{j\omega k.t}$ en notation complexe, les signaux e'i(t,r) étant lesdits signaux de référence.

**9.** Procédé selon la revendication 8, dans lequel au cours de la sous-étape (b123') on calcule p matrices $H'^{-1}(\omega k)$, respectivement par régularisation et inversion des matrices de transfert $H'(\omega k)$, et pour chaque transducteur r du réseau cible, on calcule le vecteur $E'(\omega k, r)$ par la formule :

$$E'(\omega k, r) = H'^{-1}(\omega k) . F(\omega k, j) .$$

**10.** Procédé selon la revendication 8 ou la revendication 9, dans lequel au cours de l'étape (b123'), les composantes $Fl(\omega k, r)$ du vecteur $F(\omega k, r)$ correspondant à la répartition spatiale du champ désiré à la fréquence $\omega k$, sont égales à 0 pour $l \neq r$ et égale à 1 pour $l = r$.

**11.** Procédé selon l'une quelconque des revendications 8 à 10, dans lequel au cours de la sous-étape (b222'), on assimile la paroi aberratrice, au voisinage de chaque point de focalisation r correspondant à un transducteur du réseau cible, à un filtre à réponse impulsionnelle finie, défini à chaque fréquence $\omega k$ par une amplitude $Gr(\omega k)$ et une phase $\phi_r(\omega k)$, la sous-étape (b122') comportant elle-même les sous-étapes suivantes :

(b122'1) on calcule, pour chaque fréquence $\omega k$, l'amplitude $Gr(\omega k)$ et la phase $\phi_r(\omega k)$, à partir des réponses impulsionnelles précédemment déterminées,
(b122'2) on calcule p matrices de transfert corrigées $H'(\omega k) = [H'ji(\omega k)]$, où

$$H'_{ji}(\omega_k) = H_{jl}(\omega_k) . \frac{1}{G_J(\omega_k)} e^{-j\phi_i(\omega_t)} .$$

**12.** Procédé selon la revendication 11, dans lequel au cours de la sous-étape (b122'1) on calcule, pour chaque fréquence $\omega k$, l'amplitude $Gr(\omega k)$ et la phase $\phi_r(\omega k)$, de la manière suivante :

$$Gr(\omega k) = \frac{\sqrt{\sum_{i=1}^{n} Hri(\omega k) . Hri^*(\omega k)}}{\sqrt{\sum_{i=1}^{n} Hr0,i(\omega k) . Hr0,i^*(\omega k)}}$$

$$\phi_r(\omega k) = \frac{1}{n} \sum_{i=1}^{n} \left( \arg\left( Hri(\omega k) e^{i\Delta\tau(i,r,r0)\omega k} \right) - \arg(Hr0,i(\omega k)) \right) ,$$

où :

. $H^*ri$ désigne la valeur complexe conjuguée de $Hri$,
. et $\Delta\tau(r0, r, i) = (d(r0, i) - d(r, i))/c$, $d(r, i)$ étant la distance entre le transducteur i et le point de focalisation r, et $d(r0, i)$ étant la distance entre le transducteur i et un point de focalisation particulier r0.

**13.** Procédé selon l'une quelconque des revendications précédentes, dans lequel au cours de l'étape (c), on fait suivre la sous-étape (c1) par les sous-étapes suivantes :

(c2) on fait capter par lesdits transducteurs du réseau d'imagerie des signaux $s_1(t)$ rétrodiffusés par le milieu hétérogène dissipatif,
(c3) on convolue le signal de référence émis par chaque transducteur du réseau d'imagerie avec le signal rétrodiffusé capté par ce transducteur,
(c4) puis on somme les produits de convolution ainsi obtenus,

l'étape (c) étant renouvelée pour une pluralité de points situés dans le milieu sensiblement homogène.

**14.** Procédé selon l'une quelconque des revendications précédentes, dans lequel au cours de la sous-étape (b21), la première estimation de chaque signal de référence est e'i(t,q) = ei(ts+θi(q),q0) pour chaque point de focalisation q, q0 étant l'indice d'un point de focalisation proche du point q pour lequel le signal de référence a déjà été déterminé, θi(q) étant un retard égal à une valeur δi(q)/c, où c est la célérité moyenne des ondes acoustiques dans le milieu, et δi(q) est égal à une différence entre d'une part, une distance entre le transducteur i du réseau d'imagerie et le point de focalisation q0, et d'autre part, une distance entre le transducteur i du réseau d'imagerie et le point de focalisation q,

**15.** Procédé selon l'une quelconque des revendications précédentes, dans lequel au cours de la sous-étape (b2), lorsqu'au moins certains transducteurs d'indice v du réseau d'imagerie ne sont pas directement au contact de la couche aberratrice, on corrige les signaux e'$_v$(t,q) correspondants par repropagation numérique pour simuler des transducteurs placés en contact direct avec la couche aberratrice.

**16.** Procédé selon l'une quelconque des revendications précédentes, dans lequel au cours de la sous-étape (b22), on recherche les valeurs $\alpha_1(q)$ et $\tau_1(q)$ pour maximiser le critère de cohérence C suivant :

$$C = \frac{<\left|\sum_{i=1}^{n}\alpha_i \cdot g_i(t-\tau_i,q)\right|^2>}{n.\sum_{i=1}^{n}<\left|\alpha_i \cdot g_i(t-\tau_i,q)\right|^2>} \quad ,$$

où :

. $g_i(t,q)=s_i(t)\otimes e_i(t,q)$, $\otimes$ l'opération de convolution,
. et <> représente une moyenne temporelle.

**17.** Procédé selon la revendication 16, dans lequel au cours de la sous-étape (b22), les valeurs $\tau_i(q)$ sont calculées en maximisant une fonction d'intercorrélation, pour des transducteurs voisins du réseau d'imagerie, des signaux $g_i(t,q)$ et $g_{i+1}(t,q)$.

**18.** Procédé selon la revendication 16 ou la revendication 17, dans lequel au cours de la sous-étape (b22), les valeurs $\alpha_i(q)$ sont calculées de manière à égaliser sur l'indice i l'amplitude maximale des fonctions $g_i(t,q)$.

**19.** Procédé selon l'une quelconque des revendications précédentes, dans lequel la sous-étape (b22) relative à chaque point de focalisation q est réalisée immédiatement après la sous-étape (b21) relative au même point de focalisation q.

**20.** Procédé selon l'une quelconque des revendications précédentes, dans lequel le milieu hétérogène dissipatif est constitué par le cerveau entouré par le crâne.

**21.** Procédé selon l'une quelconque des revendications précédentes, dans lequel :

- soit le réseau d'imagerie et le réseau cible sont deux réseaux distincts disposés de part et d'autre du milieu hétérogène dissipatif,
- soit tous les transducteurs appartiennent à la fois au réseau d'imagerie et au réseau cible.

**22.** Procédé selon l'une quelconque des revendications précédentes, dans lequel les ondes acoustiques sont des ondes ultrasonores.

**23.** Dispositif (1) adapté pour mettre en oeuvre un procédé selon l'une quelconque des revendications précédentes, ce dispositif comportant un nombre t supérieur à 2 de transducteurs acoustiques (T1-Tn, T'1-T'm) destinés à être fixés dans des positions prédéterminées à l'extérieur de la couche aberratrice (3), ces transducteurs étant commandés par au moins une unité centrale électronique (CPU) et formant au moins :

- un réseau d'imagerie (T1-Tn) qui regroupe un nombre n compris entre 1 et t desdits transducteurs,
- et un réseau cible (T'1-T'm) qui regroupe un nombre m compris entre 1 et t desdits transducteurs,

l'unité centrale électronique étant adaptée pour suivre les étapes suivantes :

(b)une étape d'apprentissage comprenant elle-même les sous étapes suivantes :

(b1) une sous-étape d'apprentissage de focalisation du réseau d'imagerie sur le réseau cible, étape au cours de laquelle :

(b11) on détermine des réponses impulsionnelles hri(t) du milieu hétérogène dissipatif, respectivement entre chaque transducteur i du réseau d'imagerie et plusieurs points de focalisation r situés sur la couche aberratrice en correspondance respective avec des transducteurs du réseau cible, ces réponses impulsionnelles étant mémorisées sous forme numérique avec un certain échantillonnage temporel qui détermine un nombre p de composantes fréquentielles de la réponse impulsionnelle, de fréquences respectives $\omega k$, i étant un indice compris entre 1 et n qui désigne un transducteur du réseau d'imagerie, r étant un indice compris entre 1 et m qui désigne un point de focalisation correspondant à un transducteur du réseau cible et k étant un indice compris entre 1 et p qui désigne une composante fréquentielle,

(b12) à partir de ces réponses impulsionnelles, on calcule, pour chaque point de focalisation r correspondant à un transducteur du réseau cible, un ensemble de n signaux de référence temporels e'i(t,r), i variant entre 1 et n, tels que, si la paroi aberratrice était enlevée au voisinage du point de focalisation r, l'émission de ces signaux de référence par les différents transducteurs i du réseau d'imagerie génèrerait une impulsion acoustique focalisée sur le point de focalisation r,

(b2) une sous-étape de focalisation en un nombre R de points de focalisation prédéterminés situés dans le milieu sensiblement homogène, d'indices q compris entre m+1 et m+R, cette sous-étape consistant à déterminer pour chacun de ces points de focalisation q, en s'éloignant pas à pas des points de focalisation 1 à m correspondant aux transducteurs du réseau cible, des signaux de référence e'i(t,q) à faire émettre par les différents transducteurs i du réseau d'imagerie pour générer un signal prédéterminé focalisé sur ledit point de focalisation q, les signaux de référence e'i(t,q) étant déterminés pour chaque point de focalisation q en procédant comme suit :

(b21) une première estimation de e'i(t,q), pour i allant de 1 à 4, est calculée à partir d'au moins un signal de référence e'i(t,q0), q0 étant l'indice d'au moins un point de focalisation proche du point de focalisation q pour lequel le signal de référence a déjà été déterminé, ce calcul étant fait en utilisant une célérité moyenne des ondes acoustiques dans le milieu sensiblement homogène (2),

(b22) on fait émettre par les transducteurs du réseau d'imagerie, par itérations, les estimations précédemment obtenues des signaux de référence e'i(t,q), puis on capte avec les mêmes transducteurs des signaux, $s_1$(t,q) rétrodiffusés par le milieu hétérogène dissipatif, puis on modifie pour l'itération suivante ces signaux de référence e'i(t,q) de la manière suivante :

$$e_i^{'}(t) \quad \rightarrow \quad \alpha_i(q) . e_i^{'}(i - \tau_i(q))$$

où les valeurs $\alpha_1$(q) et $\tau_i$(q) sont un facteur d'amplitude et un retard correctifs, calculés pour maximiser un critère de cohérence C entre lesdits signaux rétrodiffusés, lesdites itérations étant arrêtées lorsque le critère C atteint un seuil prédéterminé,

(b3) on mémorise les signaux de référence e'i(t,q), au moins pour q compris entre m+1 et m+R,

(c) et une étape de focalisation au cours de laquelle, pour au moins un desdits points de focalisation q, on fait émettre respectivement par les transducteurs du réseau d'imagerie, lesdits signaux de référence e'i(t,q), i étant un indice compris entre 1 et n désignant un transducteur du réseau d'imagerie.

**Claims**

1. A noninvasive method for focusing acoustic waves in a dissipative heterogeneous medium (2, 3) comprising a substantially homogeneous medium (2) surrounded at least partially by a dissipative aberrating layer (3) which generates aberrations in the propagation of the acoustic waves, the acoustic waves being emitted from outside the aberrating layer (3) and focused in the substantially homogeneous medium (2), including the following steps:

   (a) an initial positioning step during which a number t greater than 2 of acoustic transducers (T1-Tn, T'1-T'm) are fixed in predetermined positions outside the aberrating layer (3), these transducers being in contact with said aberrating layer and forming at least:

   - an imaging array (T1-Tn) which combines a number n between 1 and t of said transducers,
   - and a target array (T'1-T'm) which combines a number m between 1 and t of said transducers,

   (b) a learning step itself comprising the following substeps:

   (b1) a substep of learning to focus the imaging array on the target array, during which substep:

   (b11) impulse responses hri(t) of the dissipative heterogeneous medium are determined, respectively between each transducer i of the imaging array and a plurality of focusing points r lying on the aberrating layer (3) in respective correspondence with transducers of the target array, these impulse responses being stored in digital form with a certain time sampling which determines a number p of frequency components of the impulse response, with respective frequencies $\omega k$, i being an index between 1 and n which designates a transducer of the imaging array, r being an index between 1 and m which designates a focusing point corresponding to a transducer of the target array and k being an index between 1 and p which designates a frequency component,
   (b12) on the basis of these impulse responses, for each focusing point r corresponding to a transducer of the imaging array, a set of n reference time signals e'i(t,r) is calculated, i varying between 1 and n, such that if the aberrating wall were removed in the vicinity of the focusing point r, the emission of these reference signals by the various transducers i of the imaging array would generate a predetermined signal focused on the focusing point r,

   (b2) a substep of focusing at a number R of predetermined focusing points lying in the substantially homogeneous medium, with indices q between m+1 and m+R, this substep consisting in determining for each of these focusing points q, moving step-by-step away from the focusing points 1 to m corresponding to the transducers of the target array, reference signals e'i(t,q) to be emitted by the various transducers i of the imaging array in order to generate a pulse focused on said focusing point q, the reference signals e'i(t,q) being determined for each focusing point q by proceeding as follows:

   (b21) a first estimate of e'i(t,q), for i ranging from 1 to n, is calculated on the basis of at least one reference signal e'i(t,q0), q0 being the index of at least one focusing point close to the focusing point q for which the reference signal has already been determined, this calculation being performed by using an average speed of the acoustic waves in the substantially homogeneous medium (2),
   (b22) the transducers of the imaging array are made to emit, by iterations, the estimates previously obtained of the reference signals e'i(t,q), then signals si(t,q) back-scattered by the dissipative heterogeneous medium are picked up with the same transducers, then these reference signals e'i(t,q) are modified for the next iteration in the following way:

$$e_i^{'}(t) \quad \rightarrow \quad \alpha_i(q).e_i^{'}(t - \tau_i(q))$$

   where the values $\alpha_i(q)$ and $\tau_i(q)$ are a corrective amplitude factor and a corrective delay, which are calculated so as to maximize a coherence criterion C between said back-scattered signals, said iterations being stopped when the criterion C reaches a predetermined threshold,

   (b3) the reference signals e'i(t,q) are stored, at least for q between m+1 and m+R,

(c) and a focusing step during which, for at least one of said focusing points q, the transducers of the imaging array are made to emit said reference signals e'$_i$(t,q), i being an index between 1 and n designating a transducer of the imaging array.

2. The method as claimed in claim 1, in which during substep (b11), when at least certain transducers (T1-Tm, T'1-T'm) are in contact with an intermediate heterogeneous medium which is itself in contact with the aberrating layer, the impulse responses hri(t) are corrected by digital backpropagation in order to simulate transducers lying directly in contact with the aberrating layer.

3. The method as claimed in claim 1 or claim 2, in which substep (b12) itself includes the following substeps:

(b121) p transfer matrices H($\omega$k)=[Hri($\omega$k)] are determined, i ranging from 1 to n and r ranging from 1 to m, where Hri($\omega$k) is the value, at the frequency $\omega$k, of the Fourier transform of the impulse response hri(t),
(b122) for each focusing point r corresponding to a transducer of the target array, n components Ei($\omega$k,r) are determined, i varying between 1 and n, such that F($\omega$k,r)=H($\omega$k).E($\omega$k,r), where E($\omega$k,r)=[Ei($\omega$k,r)] is a vector with n components, F($\omega$k,r) is a vector with m components Fl($\omega$k,r), l varying between 1 and m, these m components Fl($\omega$k,r) corresponding to a desired focusing of the acoustic waves at the frequency $\omega$k on the focusing point r corresponding to a transducer of the target array,
(b123) for each focusing point r corresponding to a transducer of the target array, a vector of n time signals e(t,r)=[ei(t,r)] is deduced therefrom, i varying between 1 and n, where $e_i(t,r) = \sum_{k=1}^{p} Ei(\omega k,r).e^{j\omega k.t}$ in

complex notation, these signals ei(t,r) being adapted so that the emission of them respectively by the various transducers i of the imaging array generates an acoustic pulse focused on the focusing point r of the target array,
(b124) a substep of correcting the aberrations generated by the aberrating layer between the substantially homogeneous medium and each target transducer r, these aberrations being estimated on the basis of the measurements carried out previously, the aberrations estimated in this way being used to calculate said reference time signals e'i(t,r).

4. The method as claimed in claim 3, in which p matrices H$^{-1}$($\omega$k) are calculated during substep (b122), respectively by regularization and inversion of the transfer matrices H($\omega$k), and the vector E($\omega$k,r) is calculated for each transducer r of the target array by the formula:

$$E(\omega k,r) = H^{-1}(\omega k).F(\omega k,j).$$

5. The method as claimed in claim 3 or claim 4, in which during step (b122), the components Fl($\omega$k,r) of the vector F ($\omega$k,r) corresponding to the spatial distribution of the desired field at the frequency $\omega$k are equal to 0 for l≠r and to 1 for l=r.

6. The method as claimed in any one of claims 3 to 5, in which during substep (b124), the aberrating wall in the vicinity of each focusing point r corresponding to a transducer of the target array is assimilated to a filter, which has a finite impulse response and is defined at each frequency $\omega$k by an amplitude Gr($\omega$k) and a phase $\varphi_r$($\omega$k), substep (b124) itself including the following substeps:

(b1241) for each frequency $\omega$k, the amplitude Gr($\omega$k) and the phase $\varphi_r$($\omega$k) are calculated on the basis either of the signals ei(t,r) or of the vectors E($\omega$k,r),
(b1242) p corrected transfer matrices H'($\omega$k)=[H'ji($\omega$k)] are calculated, where

$$H'_{ji}(\omega_k) = H_{ji}(\omega_k).\frac{1}{G_j(\omega_k)}e^{-j\phi_j(\omega_k)},$$

(b1243) for each transducer r of the target array, n components E'i($\omega$k,r) are determined, i varying between 1 and n, such that F($\omega$k,r)=H'($\omega$k).E'($\omega$k,r), where E'($\omega$k,r)=[E'i($\omega$k,r)] is a vector with n components, F($\omega$k,r) is a vector with m components Fl($\omega$k,r), 1 varying between 1 and m, these m components Fl($\omega$k,r) corresponding

to a desired focusing of the acoustic waves at the frequency wk on the focusing point r corresponding to a transducer of the target array,

(b1244) for each focusing point r corresponding to a transducer of the target array, a vector of n reference time signals e'(t,r)=[e'i(t,r)] is deduced therefrom, i varying between 1 and n, where $e'_i(t,r)=\sum_{k=1}^{p} E_i(\omega k,r).e^{j\omega k.t}$ in complex notation.

7. The method as claimed in claim 6, in which during substep (b1241), the amplitude Gr($\omega$k) and the phase $\varphi_r$($\omega$k) are calculated as follows:

$$G_r(\omega k)=\frac{\sqrt{\sum_{i=1}^{n} E_i(\omega k,r0).E_i^*(\omega k,r0)}}{\sqrt{\sum_{i=1}^{n} E_i(\omega k,r).E_i^*(\omega k,r)}}$$

$$\phi_r(\omega k)=\frac{1}{n}\sum_{i=1}^{n}\left(\arg(E_i(\omega k,r0))-\arg\left(E_i(\omega k,r)e^{-j\Delta\tau(r0,r,i)\omega k}\right)\right)$$

where:

- Ei* is the complex conjugate value of Ei,
- and $\Delta\tau$(r0,r,i)=(d(r0,i)-d(r,i))/c, d(r,i) being the distance between the transducer i and the focusing point r, and d(r0,i) being the distance between the transducer i and a particular focusing point r0.

8. The method as claimed in claim 1 or claim 2, in which substep (b12) itself includes the following substeps:

(b121) p transfer matrices H($\omega$k)=[Hri($\omega$k)] are determined, i ranging from 1 to n and r ranging from 1 to m, where Hri($\omega$k) is the value, at the frequency $\omega$k, of the Fourier transform of the impulse response hri(t),
(b122') the transfer matrices H($\omega$k) are corrected in order to overcome the aberrations generated by the aberrating wall in the vicinity of each focusing point r, this correction being carried out on the basis of the impulse responses hri(t) determined previously, and corrected transfer matrices H'($\omega$k) are obtained in this way,
(b123') for each focusing point r corresponding to a transducer of the target array, n components E'i($\omega$k,r) are determined, i varying between 1 and n, such that F($\omega$k,r)=H'($\omega$k).E'($\omega$k,r), where E'($\omega$k,r)=[E'i($\omega$k,r)] is a vector with n components, F($\omega$k,r) is a vector with m components Fl($\omega$k,r) l varying between 1 and m, these m components Fl($\omega$k,r) corresponding to a desired focusing of the acoustic waves at the frequency $\omega$k on the focusing point r corresponding to a transducer of the target array,
(b124') for each focusing point r corresponding to a transducer of the target array, a vector of n time signals e' (t,r)=[e'i(t,r)] is deduced therefrom, i varying between 1 and n, where $e'_i(t,r)=\sum_{k=1}^{p} E'i(\omega k,r).e^{j\omega k.t}$ in complex notation, the signals e'i(t,r) being said reference signals.

9. The method as claimed in claim 8, in which p matrices H'⁻¹($\omega$k) are calculated during substep (b123'), respectively by regularization and inversion of the transfer matrices H'($\omega$k), and the vector E'($\omega$k,r) is calculated for each transducer r of the target array by the formula:

```
E'(ωk,r)=H'⁻¹(ωk).F(ωk,j).
```

10. The method as claimed in claim 8 or claim 9, in which during step (b123'), the components Fl(ωk,r) of the vector F (ωk,r) corresponding to the spatial distribution of the desired field at the frequency ωk are equal to 0 for l≠r and to 1 for l=r.

11. The method as claimed in any one of claims 8 to 10, in which during substep (b122'), the aberrating wall in the vicinity of each focusing point r corresponding to a transducer of the target array is assimilated to a filter, which has a finite impulse response and is defined at each frequency ωk by an amplitude Gr(ωk) and a phase $\varphi_r$(ωk), substep (b122') itself including the following substeps:

(b122'1) for each frequency ωk, the amplitude Gr(ωk) and the phase $\varphi_r$(ωk) are calculated on the basis of the impulse responses determined previously,
(b122'2) p corrected transfer matrices H'(ωk)=[H'ji(ωk)] are calculated, where

$$H'_{ji}(\omega_k) = H_{ji}(\omega_k).\frac{1}{G_j(\omega_k)}e^{-j\phi_j(\omega_k)}\ .$$

12. The method as claimed in claim 11, in which during substep (b122'1), the amplitude Gr(ωk) and the phase $\varphi_r$(ωk) are calculated for each frequency ωk in the following way:

$$G_r(\omega_k)=\frac{\sqrt{\sum_{i=1}^{n}H_{ri}(\omega_k).H_{ri}^{\bullet}(\omega_k)}}{\sqrt{\sum_{i=1}^{n}H_{r0,i}(\omega_k).H_{r0,i}^{\bullet}(\omega_k)}}$$

$$\phi_r(\omega_k)=\frac{1}{n}\sum_{i=1}^{n}\left(\arg\left(H_{ri}(\omega_k)e^{j\Delta\tau(i,r,r0)\omega_k}\right)-\arg\left(H_{r0,i}(\omega_k)\right)\right),$$

where:

- H*ri designates the complex conjugate value of Hri,
- and $\Delta\tau$(r0,r,i)=(d(r0,i)-d(r,i))/c, d(r,i) being the distance between the transducer i and the focusing point r, and d(r0,i) being the distance between the transducer i and a particular focusing point r0.

13. The method as claimed in any one of the preceding claims, in which during step (c), substep (c1) is followed by the following substeps:

(c2) said transducers of the imaging array are made to pick up signals $s_i$(t) back-scattered by the dissipative heterogeneous medium,
(c3) the reference signal emitted by each transducer of the imaging array is convoluted with the back-scattered signal picked up by this transducer,
(c4) then the convolution products obtained in this way are summed,

step (c) being repeated for a plurality of points lying in the substantially homogeneous medium.

14. The method as claimed in any one of the preceding claims, in which during substep (b21), the first estimate of each reference signal is e'i(t,q)=e'i(ts+θi(q),q0) for each focusing point q, q0 being the index of a focusing point close to the focusing point q for which the reference signal has already been determined, θi(q) being a delay equal to a value δi(q)/c, where c is the average speed of the acoustic waves in the medium, and δi(q) is equal to a difference between, on the one hand, a distance between the transducer i of the imaging array and the focusing point q0, and, on the other hand, a distance between the transducer i of the imaging array and the focusing point q.

**15.** The method as claimed in any one of the preceding claims, in which during substep (b2), when at least certain transducers with index v of the imaging array are not directly in contact with the aberrating layer, the corresponding signals $e'_v(t,q)$ are corrected by digital backpropagation in order to simulate transducers placed in direct contact with the aberrating layer.

**16.** The method as claimed in any one of the preceding claims, in which during substep (b22), the values $\alpha_i(q)$ and $\tau_i(q)$ are looked for to maximize the following coherence criterion C:

$$C = \frac{\left< \left| \sum_{i=1}^{n} \alpha_i . g_i(t - \tau_i, q) \right|^2 \right>}{n . \sum_{i=1}^{n} \left< \left| \alpha_i . g_i(t - \tau_i, q) \right|^2 \right>} \quad ,$$

where:

$\cdot \quad g_i(t,q) = s_i(t) \otimes e'_i(t,q)$ , $\otimes$ representing the convolution operation,

. and <> represents a time average.

**17.** The method as claimed in claim 16, in which during substep (b22), the values $\tau_i(q)$ are calculated by maximizing a cross-correlation function, for transducers close to the imaging array, of the signals $g_i(t,q)$ and $g_{i+1}(t,q)$.

**18.** The method as claimed in claim 16 or claim 17, in which during substep (b22), the values $\alpha_i(q)$ are calculated so as to equalize the maximum amplitude of the functions $g_i(t,q)$ on the index i.

**19.** The method as claimed in any one of the preceding claims, in which substep (b22) relating to each focusing point q is carried out immediately after substep (b21) relating to the same focusing point q.

**20.** The method as claimed in any one of the preceding claims, in which the dissipative heterogeneous medium consists of the brain surrounded by the skull.

**21.** The method as claimed in any one of the preceding claims, in which:

- either the imaging array and the target array are two separate arrays arranged on either side of the dissipative heterogeneous medium,
- or all the transducers belong both to the imaging array and to the target array.

**22.** The method as claimed in any one of the preceding claims, in which the acoustic waves are ultrasound waves.

**23.** A device (1) designed for carrying out a method as claimed in any one of the preceding claims, this device including a number t greater than 2 of acoustic transducers (T1-Tn, T'1-T'm) intended to be fixed in predetermined positions outside the aberrating layer (3), these transducers being controlled by at least one central electronic unit (CPU) and forming at least:

- an imaging array (T1-Tn) which combines a number n between 1 and t of said transducers,
- and a target array (T'1-T'm) which combines a number m between 1 and t of said transducers,

the central electronic unit being designed to follow the following steps:

(b) a learning step itself comprising the following substeps:

(b1) a substep of learning to focus the imaging array on the target array, during which substep:

(b11) impulse responses hri(t) of the dissipative heterogeneous medium are determined, respectively between each transducer i of the imaging array and a plurality of focusing points r lying on the aberrating layer in respective correspondence with transducers of the target array, these impulse responses being stored in digital form with a certain time sampling which determines a number p of frequency components of the impulse response, with respective frequencies $\omega k$, i being an index between 1 and n which designates a transducer of the imaging array, r being an index between 1 and m which designates a focusing point corresponding to a transducer of the target array and k being an index between 1 and p which designates a frequency component,

(b12) on the basis of these impulse responses, for each focusing point r corresponding to a transducer of the imaging array, a set of n reference time signals e'i(t,r) is calculated, i varying between 1 and n, such that if the aberrating wall were removed in the vicinity of the focusing point r, the emission of these reference signals by the various transducers i of the imaging array would generate an acoustic pulse focused on the focusing point r,

(b2) a substep of focusing at a number R of predetermined focusing points lying in the substantially homogeneous medium, with indices q between m+1 and m+R, this substep consisting in determining for each of these focusing points q, moving step-by-step away from the focusing points 1 to m corresponding to the transducers of the target array, reference signals e'i(t,q) to be emitted by the various transducers i of the imaging array in order to generate a predetermined signal focused on said focusing point q, the reference signals e'i(t,q) being determined for each focusing point q by proceeding as follows:

(b21) a first estimate of e'i(t,q), for i ranging from 1 to 4, is calculated on the basis of at least one reference signal e'i(t,q0), q0 being the index of at least one focusing point close to the focusing point q for which the reference signal has already been determined, this calculation being performed by using an average speed of the acoustic waves in the substantially homogeneous medium (2),

(b22) the transducers of the imaging array are made to emit, by iterations, the estimates previously obtained of the reference signals e'i(t,q), then signals $s_i(t,q)$ back-scattered by the dissipative heterogeneous medium are picked up with the same transducers, then these reference signals e'i(t,q) are modified for the next iteration in the following way:

$$e_i^{'}(t) \quad \rightarrow \quad \alpha_i(q) \cdot e_i^{'}(t - \tau_i(q))$$

where the values $\alpha_i(q)$ and $\tau_i(q)$ are a corrective amplitude factor and a corrective delay, which are calculated so as to maximize a coherence criterion C between said back-scattered signals, said iterations being stopped when the criterion C reaches a predetermined threshold,

(b3) the reference signals e'i(t,q) are stored, at least for q between m+1 and m+R,

(c) and a focusing step during which, for at least one of said focusing points q, the transducers of the imaging array are made to emit said reference signals e'i(t,q), i being an index between 1 and n designating a transducer of the imaging array.

## Patentansprüche

1. Nichtinvasives Verfahren zum Fokussieren von Schallwellen in ein dissipatives heterogenes Medium (2,3), das ein im wesentlichen homogenes Medium (2) aufweist, das zumindest teilweise von einer dissipativen Aberrationsschicht (3) umgeben ist, die bei der Fortpflanzung von Schallwellen Abberationen erzeugt, wobei die Schallwellen von außerhalb der Aberrationsschicht (3) ausgesandt werden und in das im wesentlichen homogene Medium (2) fokussiert werden, mit den folgenden Schritten:

(a) einem Anfangsschritt des Positionierens, im Verlaufe dessen man eine Anzahl t größer 2 von akustischen Wandlern (T1-Tn, T'1-T'm) an vorgegebenen Positionen außerhalb der Aberrationsschicht (3) anbringt, wobei diese Wandler in Kontakt mit der Aberrationsschicht sind und mindestens

- ein Abbildungsnetz (T1-Tn), das eine Anzahl n zwischen 1 und t der Wandler zusammenfaßt,
- und ein Zielnetz (T'1-T'm), das eine Anzahl m zwischen 1 und t der Wandler zusammenfaßt, bilden,

(b) einem Schritt des Lernens, der seinerseits die folgenden Teilschritte aufweist:

(b1) einen Teilschritt des Lernens der Fokussierung des Abbildungsnetzes auf das Zielnetz, wobei im Verlaufe dieses Teilschritts

(b11) Impulsantworten hri(t) des heterogenen dissipativen Mediums jeweils zwischen jedem Wandler i des Abbildungsnetzes und mehreren an der Aberrationsschicht (3) gelegenen Fokussierungspunkten r, die jeweils Wandlern des Zielnetzes entsprechen, bestimmt werden, wobei diese Impulsantworten in digitaler Form mit einer gewissen zeitlichen Abtastung gespeichert werden, die eine Anzahl p von Frequenzkomponenten der Impulsantworten für jeweilige Frequenzen $\omega k$ festlegt, wobei i ein Index zwischen 1 und n ist, der einen Wandler des Abbildungsnetzes bezeichnet, r ein Index zwischen 1 und m ist, der einen Fokussierungspunkt, der einem Wandler des Zielnetzes entspricht, bezeichnet, und k ein Index zwischen 1 und p ist, der eine Frequenzkomponente bezeichnet,
(b12) ausgehend von diesen Impulsantworten für jeden Fokussierungspunkt r, der einem Wandler des Zielnetzes entspricht, eine Gesamtheit von n Referenzzeitsignalen e'i(t,r), mit i zwischen 1 und n, derart berechnet werden, daß, wenn die Aberrationswand in der Nähe des Fokussierungspunkts r weggenommen wäre, das Aussenden dieser Referenzsignale aus den verschiedenen Wandlern i des Abbildungsnetzes ein auf den Fokussierungspunkt r fokussiertes vorgegebenes Signal erzeugen

würde, (b2) einen Teilschritt der Fokussierung auf eine Anzahl R von vorgegebenen Fokussierungspunkten, die in dem im wesentlichen homogenen Medium gelegen sind, der Indizes q zwischen m+1 und m+R, wobei dieser Teilschritt darin besteht, daß man für jeden dieser Fokussierungspunkte q, indem man sich schrittweise von den Fokussierungspunkten 1 bis m, die den Wandlern des Zielnetzes entsprechen, entfernt, Referenzsignale e'i(t,q) bestimmt, die man von den verschiedenen Wandlern i des Abbildungsnetzes aussenden läßt, um einen auf den Fokussierungspunkt q fokussierten Impuls zu erzeugen, wobei die Referenzsignale e'i(t,q) für jeden Fokussierungspunkt q bestimmt werden, indem man wie folgt vorgeht:

(b21) ausgehend von mindestens einem Referenzsignal e'i(t,q0) wird eine erste Schätzung von e'i(t, q), mit i von 1 bis n, berechnet, wobei q0 der Index mindestens eines Fokussierungspunkts in der Nähe des Fokussierungspunkts q ist, für welchen das Referenzsignal bereits bestimmt worden ist, wobei diese Berechnung unter Verwendung einer durchschnittlichen Fortpflanzungsgeschwindigkeit der Schallwellen in dem im wesentlichen homogenen Medium (2) durchgeführt
wird (b22) man von den Wandlern des Abbildungsnetzes iterativ die vorstehend gewonnenen Schätzungen der Referenzsignale e'i(t,q) aussenden läßt, man dann Signale si(t,q), die von dem heterogenen dissipativen Medium zurück gestreut worden sind, mit den gleichen Wandlern erfaßt, man dann für die nächste Iteration diese Referenzsignale e'i(t,q) wie folgt modifiziert:

$$e'_i(t) \; \rightarrow \; a_i(q) \bullet e'_i(t - \tau_i(q))$$

wobei die Werte $\alpha_i(g)$ und $\tau_i(q)$ ein korrigierender Amplitudenfaktor und eine korrigierende Verzögerung sind, die berechnet werden, um ein Kohärenzkriterium C zwischen den zurückgestreuten Signalen zu maximieren, wobei die Iterationen beendet werden, wenn das Kriterium C eine vorgegebene Schwelle erreicht,

(b3) eine Speicherung der Referenzsignale e'i(t,q) zumindest für q zwischen m+1 und m+R,

(c) und einen Fokussierungsschritt, im Verlaufe dessen man für mindestens einen der Fokussierungspunkte q die Referenzsignale e'i(t,q) jeweils aus den Wandlern des Abbildungsnetzes aussenden läßt, wobei i ein Index zwischen 1 und n ist, der einen Wandler des Abbildungsnetzes bezeichnet.

2. Verfahren nach Anspruch 1, in welchem man im Verlaufe des Teilschritts (b11) für den Fall, daß mindestens einige Wandler (T1-Tm, T'1-T'm) in Kontakt mit einem homogenen Zwischenmedium sind, das seinerseits in Kontakt mit der Aberrationsschicht ist, die Impulsantworten hri(t) durch rechnerische Backpropagation korrigiert, um Wandler zu simulieren, die in direktem Kontakt mit der Aberrationsschicht angeordnet sind.

3. Verfahren nach Anspruch 1 oder Anspruch 2, in welchem der Teilschritt (b12) seinerseits die folgenden Teilschritte aufweist:

(b121) man bestimmt p Übertragungsmatrizen H(ωk)=[Hri(ωk)], mit i von 1 bis n und r von 1 bis m, wobei Hri(ωk) der Wert der Fouriertransformierten der Impulsantwort hri(t) für die Frequenz ωk ist,

(b122) man bestimmt für jeden Fokussierungspunkt r, der einem Wandler des Zielnetzes entspricht, n Komponenten Ei(ωk,r), mit i von 1 bis n, derart, daß F(ωk,r)=H(ωk)•E(ωk,r), wobei E(ωk,r)=[Ei(ωk,r)] ein Vektor mit n Komponenten ist und F(ωk,r) ein Vektor mit m Komponenten Fl(ωk,r), mit 1 von 1 bis m, ist, wobei diese m Komponenten Fl(ωk,r) einer gewünschten Fokussierung der Schallwellen der Frequenz ωk auf den Fokussierungspunkt r, der einem Wandler des Zielnetzes entspricht, entsprechen,

(b123) man folgert daraus für jeden Fokussierungspunkt r, der einem Wandler des Zielnetzes entspricht, einen Vektor aus n Zeitsignalen e(t,r)=[ei(t,r)], mit i von bis n,

mit

$$ei(t,r) = \sum_{k=1}^{p} Ei(\omega k,r) \cdot e^{j\omega kt}$$

in komplexer Darstellung,

wobei diese Signale ei(t,r) so angepaßt sind, daß ihr Aussenden jeweils aus verschiedenen Wandlern i des Abbildungsnetzes einen akustischen Impuls erzeugt, der auf den Fokussierungspunkt r des Zielnetzes fokussiert ist,

(b124) einen Teilschritt zum Korrigieren der von der Aberrationsschicht zwischen dem im wesentlichen homogenen Medium und jedem Zielwandler r erzeugten Aberrationen, wobei diese Aberrationen auf der Basis von vorab durchgeführten Messungen geschätzt werden, wobei die auf diese Weise geschätzten Aberrationen verwendet werden, um die Referenzzeitsignale e'i(t,r) zu berechnen.

4.  Verfahren nach Anspruch 3, in welchem man im Verlaufe des Teilschritts (b122) p Matrizen $H^{-1}(\omega k)$ jeweils durch Regularisierung und Inversion der Übertragungsmatrizen H(ωk) berechnet und man für jeden Wandler r des Zielnetzes den Vektor E(ωk,r) mit Hilfe der folgenden Formel berechnet:

$$E(\omega k,r) = H^{-1}(\omega k) \bullet F(\omega k,j)$$

5.  Verfahren nach Anspruch 3 oder Anspruch 4, in welchem im Verlaufe des Schritts (b122) die Komponenten Fl(ωk, r) des Vektors F(ωk,r), der der räumlichen Verteilung des gewünschten Felds bei der Frequenz ωk entspricht, für l≠r gleich 0 und für 1=r gleich 1 sind.

6.  Verfahren nach einem der Ansprüche 3 bis 5, in welchem man im Verlaufe des Teilschritts (b124) die Aberrationswand in der Nähe jedes Fokussierungspunkts r, der einem Wandler des Zielnetzes entspricht, mit einem Filter endlicher Impulsantwort gleichsetzt, das für jede Frequenz ωk durch eine Amplitude Gr(ωk) und eine Phase Φr(ωk) definiert ist, wobei der Teilschritt (b124) seinerseits die folgenden Teilschritte aufweist:

(b1241) man berechnet für jede Frequenz ωk die Amplitude Gr(ωk) und die Phase Φr(ωk) ausgehend entweder von den Signalen ei(t,r) oder von den Vektoren E(ωk,r),

(b1242) man berechnet p korrigierte Übertragungsmatrizen H'(ωk)=[H'ji(ωk)], mit

$$H'_{ji}(\omega_k) = H_{ji}(\omega_k) . \frac{1}{G_j(\omega_k)} e^{-j\phi_{,}(\omega_i)} ,$$

(b1243) man bestimmt für jeden Wandler r des Zielnetzes n Komponenten E'i(ωk,r), mit i von 1 bis n, derart, daß F(ωk,r)=H'(ωk,r)•**E'**(ωk,r), wobei E'(ωk,r)=[E'i(ωk,r)] ein Vektor mit n Komponenten und F(ωk,r) ein Vektor mit m Komponenten Fl(ωk,r), mit l von 1 bis m, ist, wobei diese m Komponenten Fl(ωk,r) einer gewünschten Fokussierung der Schallwellen der Frequenz ωk auf den Fokussierungspunkt r, der einem Wandler des Zielnetzes entspricht, entsprechen,

(b1244) man folgert daraus für jeden Fokussierungspunkt r, der einem Wandler des Zielnetzes entspricht, einen Vektor aus n Referenzzeitsignalen e'(t,r)=[e'i(t,r)], mit i von 1 bis n, mit

$$e'i(t,r) = \sum_{k=1}^{p} Ei(\omega k, r) \cdot e^{j\omega kt}$$

in komplexer Darstellung.

**7.** Verfahren nach Anspruch 6, in welchem man im Verlaufe des Teilschritts (b1241) die Amplitude Gr($\omega$k) und die Phase $\Phi$r($\omega$k) wie folgt berechnet:

$$G_r(\omega_k) = \frac{\sqrt{\sum_{i=1}^{n} E_i(\omega_k, r0).E_i^*(\omega_k, r0)}}{\sqrt{\sum_{i=1}^{n} E_i(\omega_k, r).E_i^*(\omega_k, r)}}$$

$$\phi_r(\omega_k) = \frac{1}{n} \sum_{i=1}^{n} \left( \arg\left(E_i(\omega_k, r0)\right) - \arg\left(E_i(\omega_k, r)e^{-i\Delta\tau(r0, r, i)\omega_k}\right) \right)$$

wobei

- Ei* der konjugiert komplexe Wert von Ei ist,
- und $\Delta\tau$(r0,r,i)=(d(r0,i)-d(r,i))/c ist, wobei d(r,i) der Abstand zwischen dem Wandler i und dem Fokussierungspunkt r ist und d(r0,i) der Abstand zwischen dem Wandler i und einem speziellen Fokussierungspunkt r0 ist.

**8.** Verfahren nach Anspruch 1 oder Anspruch 2, in welchem der Teilschritt (b12) seinerseits die folgenden Teilschritte aufweist:

(b121) man bestimmt p Übertragungsmatrizen H($\omega$k)=[Hri($\omega$k)], mit i von 1 bis n und r von 1 bis m, wobei Hri ($\omega$k) der Wert der Fouriertransformierten der Impulsantwort hri(t) für die Frequenz $\omega$k ist,
(b122') man korrigiert die Übertragungsmatrizen H($\omega$k), um die von der Aberrationswand in der Nähe jedes Fokussierungspunkts r erzeugten Aberrationen zu eliminieren, wobei diese Korrektur ausgehend von den zuvor bestimmten Impulsantworten hri(t) durchgeführt wird, und man gewinnt auf diese Weise korrigierte Übertragungsmatrizen H'($\omega$k),
(b123') man bestimmt für jeden Fokussierungspunkt r, der einem Wandler des Zielnetzes entspricht, n Komponenten E'i($\omega$k,r), mit i von 1 bis n, derart, daß F($\omega$k,r)=H'($\omega$k)•E'($\omega$k,r), wobei E'($\omega$k ,r)=[E'i($\omega$k,r)] ein Vektor mit n Komponenten ist und F($\omega$k) ein Vektor mit m Komponenten Fl($\omega$k,r), mit 1 von 1 bis m, ist, wobei diese m Komponenten Fl($\omega$k,r) einer gewünschten Fokussierung der Schallwellen der Frequenz $\omega$k auf den Fokussierungspunkt r, der einem Wandler des Zielnetzes entspricht, entsprechen,
(b124') man folgert daraus für jeden Fokussierungspunkt r, der einem Wandler des Zielnetzes entspricht, einen Vektor aus n Zeitsignalen e'(t,r)=[e'i(t,r)], mit i von bis n,
mit

$$e'i(t,r) = \sum_{k=1}^{p} Ei(\omega k, r) \cdot e^{j\omega kt}$$

in komplexer Darstellung,
wobei e'i(t,r) die Referenzsignale sind.

**9.** Verfahren nach Anspruch 8, in welchem man im Verlaufe des Teilschritts (b123') p Matrizen H'$^{-1}$($\omega$k) jeweils durch Regularisierung und Inversion der Übertragungsmatrizen H'($\omega$k) berechnet und man für jeden Wandler r des Zielnetzes den Vektor E'(ωk,r) mit Hilfe der folgenden Formel berechnet:

$$\mathrm{E}'(\omega k,r) = \mathrm{H}'^{-1}(\omega k) \bullet \mathrm{F}(\omega k, j).$$

**10.** Verfahren nach Anspruch 8 oder Anspruch 9, in welchem im Verlaufe des Schritts (b123') die Komponenten Fl(ωk, r) des Vektors F(ωk,r), der der räumlichen Verteilung des gewünschten Felds bei der Frequenz ωk entspricht, gleich 0 sind, für l≠r, und gleich 1 sind, für 1=r.

**11.** Verfahren nach einem der Ansprüche 8 bis 10, in welchem man im Verlaufe des Teilschritts (b122') die Aberrationswand in der Nähe jedes Fokussierungspunkts r, der einem Wandler des Zielnetzes entspricht, mit einem Filter endlicher Impulsantwort gleichsetzt, das für jede Frequenz ωk durch eine Amplitude Gr(ωk) und eine Phase Φr(ωk) definiert ist, wobei der Teilschritt (b122') seinerseits die folgenden Teilschritte aufweist:

(b122'1) man berechnet für jede Frequenz ωk die Amplitude Gr(ωk) und die Phase Φr(ωk) ausgehend von den zuvor bestimmten Impulsantworten,
(b122'2) man berechnet p korrigierte Übertragungsmatrizen H'(ωk)=[H'ji(ωk)], mit

$$H'_{ji}(\omega_k) = H_{ji}(\omega_k) \cdot \frac{1}{G_j(\omega_k)} e^{-j\phi_i(\omega_k)}.$$

**12.** Verfahren nach Anspruch 11, im welchem man im Verlaufe des Teilschritts (b122'1) für jede Frequenz ωk die Amplitude Gr(ωk) und die Phase Φr(ωk) wie folgt berechnet:

$$G_r(\omega_k) = \frac{\sqrt{\sum_{i=1}^{n} H_{ri}(\omega_k) . H_{ri}^{*}(\omega_k)}}{\sqrt{\sum_{i=1}^{n} H_{r0.i}(\omega_k) . H_{r0.i}^{*}(\omega_k)}}$$

$$\phi_r(\omega_k) = \frac{1}{n} \sum_{i=1}^{n} \left( \arg\left( H_{ri}(\omega_k) e^{j\Delta\tau(i,r,r0)\omega_k} \right) - \arg\left( H_{r0.i}(\omega_k) \right) \right),$$

wobei:

- H*ri den konjugiert komplexen Wert von Hri bezeichnet,
- und Δτ(r0,r,i)=(d(r0,i)-d(r,i))/c ist, wobei d(r,i) der Abstand zwischen dem Wandler i und dem Fokussierungspunkt r ist und d(r0, i) der Abstand zwischen dem Wandler i und einem speziellen Fokussierungspunkt r0 ist.

**13.** Verfahren nach einem der vorstehenden Ansprüche, in welchem sich im Verlaufe des Schritts (c) nach dem Teilschritt (c1) die folgenden Teilschritte anschließen:

(c2) man läßt von den Wandlern des Abbildungsnetzes Signale si(t) erfassen, die von dem heterogenen dissipativen Medium zurückgestreut worden sind,
(c3) man faltet das von jedem Wandler des Abbildungsnetzes ausgesandte Referenzsignal mit dem von diesem Wandler erfaßten zurückgestreuten Signal,
(c4) dann addiert man die auf diese Weise gewonnenen Faltungsprodukte,

wobei der Schritt (c) für mehrere in dem im wesentlichen homogenen Medium gelegene Punkten wiederholt wird.

**14.** Verfahren nach einem der vorstehenden Ansprüche, in welchem im Verlaufe des Teilschritts (b21) die erste Schätzung jedes Referenzsignals e'$_i$(t,q) = e'$_i$(ts+$\Theta$i(q),q0) für jeden Fokussierungspunkt q ist, wobei q0 der Index eines Fokussierungspunkts in der Nähe des Punkts q ist, für welchen das Referenzsignal bereits bestimmt worden ist, $\Theta$i(q) eine Verzögerung gleich einem Wert $\delta$i(q)/c ist, wobei c die durchschnittliche Fortpflanzungsgeschwindigkeit der Schallwellen in dem Medium ist und $\delta$i(q) gleich einer Differenz zwischen einerseits einem Abstand zwischen dem Wandler i des Abbildungsnetzes und dem Fokussierungspunkt q0 und andererseits einem Abstand zwischen dem Wandler i des Abbildungsnetzes und dem Fokussierungspunkt q ist.

**15.** Verfahren nach einem der vorstehenden Ansprüche, in welchem man im Verlaufe des Teilschritts (b2) für den Fall, daß mindestens einige Wandler des Indexes v des Abbildungsnetzes nicht in direktem Kontakt mit der Aberrationsschicht sind, die entsprechenden Signale e'$_v$(t,q) durch Backpropagation korrigiert, um Wandler zu simulieren, die in direktem Kontakt mit der Aberrationsschicht angeordnet sind.

**16.** Verfahren nach einem der vorstehenden Ansprüche, in welchem man im Verlaufe des Teilschritts (b22) die Werte $\alpha_i$(q) und $\tau_i$(q) ermittelt, um das folgende Kohärenzkriterium C zu maximieren:

$$C = \frac{<\left|\sum_{i=1}^{n}\alpha_i \cdot g_i(t-\tau_i,q)\right|^2>}{n.\sum_{i=1}^{n}<\left|\alpha_i \cdot g_i(t-\tau_i,q)\right|^2>}$$

mit:

$$g_i(t,q) = s_i(t) \otimes e_i{'}(t,q),$$

wobei $\otimes$ für den Faltungsoperator steht, und
< > für einen zeitlichen Mittelwert steht.

**17.** Verfahren nach Anspruch 16, in welchem im Verlaufe des Teilschritts (b22) die Werte $\tau_i$(q) berechnet werden, indem für benachbarte Wandler des Abbildungsnetzes eine Korrelationsfunktion der Signale g$_i$(t,q) und g$_{i+1}$(t,q) maximiert wird.

**18.** Verfahren nach Anspruch 16 oder Anspruch 17, im welchem im Verlaufe des Teilschritts (b22) die Werte $\alpha_i$(q) berechnet werden, um über den Index i die maximale Amplitude der Funktionen g$_i$(t,q) abzugleichen.

**19.** Verfahren nach einem der vorstehenden Ansprüche, in welchem der Teilschritt (b22) bezogen auf jeden Fokussierungspunkt q sofort nach dem Teilschritt (b21) bezogen auf den gleichen Fokussierungspunkt q durchgeführt wird.

**20.** Verfahren nach einem der vorstehenden Ansprüche, in welchem das heterogene dissipative Medium aus dem von dem Schädel umgebenen Gehirn gebildet wird.

**21.** Verfahren nach einem der vorstehenden Ansprüche, in welchem

- entweder das Abbildungsnetz und das Zielnetz zwei unterschiedliche Netze sind, die auf der einen und der anderen Seite des heterogenen dissipativen Mediums angeordnet sind,
- oder alle Wandler zugleich dem Abbildungsnetz und dem Zielnetz zugeordnet sind.

**22.** Verfahren nach einem der vorstehenden Ansprüche, in welchem die Schallwellen Ultraschallwellen sind.

**23.** Vorrichtung (1) zur Durchführung eines Verfahrens nach einem der vorstehenden Ansprüche, wobei diese Vorrichtung eine Anzahl t größer 2 von akustischen Wandlern (T1-Tn, T'1-T'm) aufweist, die an vorgegebenen Positionen außerhalb der Aberrationsschicht (3) anzubringen sind, wobei diese Wandler von mindestens einer elektronischen Zentraleinheit (CPU) gesteuert werden und mindestens

- ein Abbildungsnetz (T1-Tn), das eine Anzahl n zwischen 1 und t der Wandler zusammenfaßt,
- und ein Zielnetz (T'1-T'm), das eine Anzahl m zwischen 1 und t der Wandler zusammenfaßt,

bilden,
wobei die elektronische Zentraleinheit angepaßt ist, die folgenden Schritte durchzuführen:

(b) einen Schritt des Lernens, der seinerseits die folgenden Teilschritte aufweist:

(b1) einen Teilschritt des Lernens der Fokussierung des Abbildungsnetzes auf das Zielnetz, wobei im Verlaufe dieses Teilschritts

(b11) die Impulsantworten hri(t) des heterogenen dissipativen Mediums jeweils zwischen jedem Wandler i des Abbildungsnetzes und mehreren an der Aberrationsschicht gelegenen Fokussierungspunkten r, die jeweils Wandlern des Zielnetzes entsprechen, bestimmt werden, wobei diese Impulsantworten in digitaler Form mit einer gewissen zeitlichen Abtastung gespeichert werden, die eine Anzahl p von Frequenzkomponenten der Impulsantworten für jeweilige Frequenzen $\omega k$ festlegt, wobei i ein Index zwischen 1 und n ist, der einen Wandler des Abbildungsnetzes bezeichnet, r ein Index zwischen 1 und m ist, der einen Fokussierungspunkt, der einem Wandler des Zielnetzes entspricht, bezeichnet, und k ein Index zwischen 1 und p ist, der eine Frequenzkomponente bezeichnet,
(b12) ausgehend von diesen Impulsantworten für jeden Fokussierungspunkt r, der einem Wandler des Zielnetzes entspricht, eine Gesamtheit von n Referenzzeitsignalen e'i(t,r), mit i zwischen 1 und n, derart berechnet werden, daß, wenn die Aberrationswand in der Nähe des Fokussierungspunkts weggenommen wäre, das Aussenden dieser Referenzsignale aus den verschiedenen Wandlern i des Abbildungsnetzes einen auf den Fokussierungspunkt r fokussierten akustischen Impuls erzeugen würde,

(b2) einen Teilschritt der Fokussierung auf eine Anzahl R von vorgegebenen Fokussierungspunkten, die in dem im wesentlichen homogenen Medium gelegen sind, der Indizes q zwischen m+1 und m+R, wobei dieser Teilschritt darin besteht, daß man für jeden dieser Fokussierungspunkte q, indem man sich schrittweise von den Fokussierungspunkten 1 bis m, die den Wandlern des Zielnetzes entsprechen, entfernt, Referenzsignale e'i(t,q) bestimmt, die man von den verschiedenen Wandlern i des Abbildungsnetzes aussenden läßt, um einen auf den Fokussierungspunkt q fokussierten Impuls zu erzeugen, wobei die Referenzsignale e'i(t,q) für jeden Fokussierungspunkt q bestimmt werden, indem man wie folgt vorgeht:

(b21) ausgehend von mindestens einem Referenzsignal e'i(t,q0) wird eine erste Schätzung von e'i(t,q), mit i von 1 bis 4, berechnet, wobei q0 der Index mindestens eines Fokussierungspunkts in der Nähe des Fokussierungspunkts q ist, für welchen das Referenzsignal bereits bestimmt worden ist, wobei diese Berechnung unter Verwendung einer durchschnittlichen Fortpflanzungsgeschwindigkeit der Schallwellen in dem im wesentlichen homogenen Medium (2) durchgeführt wird,
(b22) man von den Wandlern des Abbildungsnetzes iterativ die vorstehend gewonnenen Schätzungen der Referenzsignale e'i(t,q) aussenden läßt, man dann Signale si(t,q), die von dem heterogenen dissipativen Medium zurückgestreut worden sind, mit den gleichen Wandlern erfaßt, man dann für die nächste Iteration diese Referenzsignale e'i(t,q) wie folgt modifiziert:

$$e'_i(t) \ \rightarrow \ \alpha_i(q) \bullet e'_i(t - \tau_i(q))$$

wobei die Werte $\alpha_i(q)$ und $\tau_i(q)$ ein korrigierender Amplitudenfaktor und eine korrigierende Verzögerung sind, die berechnet werden, um ein Kohärenzkriterium C zwischen den zurückgestreuten Signalen zu maximieren, wobei die Iterationen beendet werden, wenn das Kriterium C eine vorgegebene Schwelle erreicht,

(b3) man die Referenzsignale e'i(t,q) zumindest für q zwischen m+1 und m+R speichert,

(c) und einen Fokussierungsschritt, im Verlaufe dessen man für mindestens einen der Fokussierungspunkte q die Referenzsignale e'i(t,q) jeweils aus den Wandlern des Abbildungsnetzes aussenden läßt, wobei i ein Index zwischen 1 und n ist, der einen Wandler des Abbildungsnetzes bezeichnet.

## RÉFÉRENCES CITÉES DANS LA DESCRIPTION

**Documents brevets cités dans la description**

- WO 0055616 A

**Littérature non-brevet citée dans la description**

- **C.DORME ; M. FINK.** Ultrasonic beam steering through inhomogeneous layers with a time reversal mirror. *IEEE Transactions Ultrasonics, Ferroelectric and Frequency Control,* Janvier 1996, vol. 43 (1), 167-175
- Focusing and steering through absorbing and aberrating layers : Application to ultrasonic propagation through the skull. *Journal of Acoustical Society of America,* Mai 1998, vol. 103 (5), 2403-2410
- **LIU, D.-L. ; WAAG, R. C.** Propagation and backpropagation for ultrasonic wavefront design. *IEEE Trans. on Ultras. Ferro. and Freq. Contr.,* 1997, vol. 44 (1), 1-13
- **MALLART et al.** The Van Cittert-Zernike theorem in pulse echo measurements. *J. Acoust. Soc. Am.,* Novembre 1991, vol. 90 (5), 2716-2727
- *J. Acoust. Soc. Am.,* Décembre 1994, vol. 96 (6), 3721-3732